# EUROPEAN PATENT SPECIFICATION

(11) **EP 2 754 711 B1**
(45) Date of publication and mention of the grant of the patent: **01.02.2017**
(21) Application number: 13425004.2
(22) Date of filing: 10.01.2013
(51) Int. Cl.: C12N 9/02, C12N 15/82

(54) **Co gene MdCo31 of the 'Wijcik' mutant of Malus x domestica Borkh and plants with controlled tree architecture genetically transformed by introduction of this gene**
Co-Gen MdCo31 des Wijcik-Mutanten von Malus x domestica Borkh und durch die Induzierung dieses Gens genetisch transformierte Pflanzen mit kontrollierter Baumarchitektur
Gène Co Mdco31 du mutant 'wijcik' de Malus x domestica Borkh et plantes à architecture arborescente contrôlée génétiquement transformées par introduction de ce gène

(43) Date of publication of application: 16.07.2014
(73) Proprietor: Fondazione Edmund Mach, 38010 San Michele All'adige (TN) (IT)
(72) Inventor: Wolters, Pieter Jacobus, 6707 BH Wageningen (NL); Baldi, Paolo, 38057 Pergine Valsugana (TN) (IT); Schouten, Henk, 6721 HM Bennekom (NL); Velasco, Riccardo, 38068 Rovereto (TN) (IT); Si Ammour, Azeddine, 38121 Trento (IT)
(74) Representative: Bonini, Ercole

(56) References cited:
- EP-A1- 2 383 344
- BALDI PAOLO ET AL: "Genetic and physical characterisation of the locus controlling columnar habit in apple (Malus x domestica Borkh.)", MOLECULAR BREEDING, vol. 31, no. 2, 3 November 2012 (2012-11-03), pages 429-440, XP002698328,
- TUANHUI BAI ET AL: "Fine genetic mapping of thelocus controlling columnar growth habit in apple", MOLECULAR GENETICS AND GENOMICS, SPRINGER, BERLIN, DE, vol. 287, no. 5, 17 April 2012 (2012-04-17), pages 437-450, XP035046605, ISSN: 1617-4623, DOI: 10.1007/S00438-012-0689-5
- MORIYA SHIGEKI ET AL: "Development of a Marker-assisted Selection System for Columnar Growth Habit in Apple Breeding", JOURNAL OF THE JAPANESE SOCIETY FOR HORTICULTURAL SCIENCE, vol. 78, no. 3, July 2009 (2009-07), pages 279-287, XP002698329,
- YI-KE TIAN ET AL: "Mapping Co, a gene controlling the columnar phenotype of apple, with molecular markers", EUPHYTICA, KLUWER ACADEMIC PUBLISHERS, DO, vol. 145, no. 1-2, 1 September 2005 (2005-09-01), pages 181-188, XP019240970, ISSN: 1573-5060, DOI: 10.1007/S10681-005-1163-9
- HENRYK FLACHOWSKY ET AL: "Overexpression of LEAFY in apple leads to a columnar phenotype with shorter internodes", PLANTA ; AN INTERNATIONAL JOURNAL OF PLANT BIOLOGY, SPRINGER, BERLIN, DE, vol. 231, no. 2, 10 November 2009 (2009-11-10), pages 251-263, XP019781413, ISSN: 1432-2048
- CLEMENS KROST ET AL: "The transcriptomes of columnar and standard type apple trees () A comparative study", GENE, ELSEVIER, AMSTERDAM, NL, vol. 498, no. 2, 27 January 2012 (2012-01-27), pages 223-230, XP028474649, ISSN: 0378-1119, DOI: 10.1016/J.GENE.2012.01.078 [retrieved on 2012-02-13]

### SEQUENCE LISTING

<110> FONDAZIONE EDMUND MACH
<120> Co gene MdCo31 of the 'wijcik' mutant of Malus x domestica Borkh and plants with controlled tree architecture genetically transformed by introduction of this gene
<130> 55.352 FEM
<160> 129
<170> PatentIn version 3.5
<210> 1
   <211> 1020
   <212> DNA
   <213> Malus x domestica Borkh
<400> 1
   atggagacat tagaccagaa tcttgtttca agctggtttg atgttcaatc tgttccccaa      60
   acctttgtcc acccacctga aaagcggccc ggtaacagta ttgacgttcc tccgtgcaag     120
   aacattccgg tagttgatct tggcagccgt gatcgcagtc acacaattca acaaatttcc     180
   aaggctagcc aagacttcgg atttttccag gtcttcaatc atggggtttg caagaagttg     240
   attgatgaca taatgagtat ttccaaggag tttcataaaa tgccccgaaa agataagata     300
   attgaaggct caaaggaccc tagtggaaga tgcaagttct acacaagcag tgaaaactat     360
   gcgaatgaag aggttcacta ttggcgagat gcattgactc accctgctca ttcttctgaa     420
   aactacatgc agtttttgcc tcaaaaacct actcaatacc gagaagtttt caaggcatac     480
   gtggatgagg taagaaacat gggttctatg attttggaga tgcttgctga agggttggga     540
   ctgagcaaag agttcttcaa tggtggactt agtgaaaatc caacgctgct gtccaaccat     600
   tatccgccat gcccagatcc tactttaacc ttgggattaa cgaaacaccg cgatccgagc     660
   ctcataacca ttttacttca agattcagaa ggacttcaag tcttcaaaga tgggaattgg     720
   attggtgttg agcctatttc tagtggcttt gttgttaaca taggctacgt catgcagatg     780
   atcagcaact cgaagtttaa aggcgctgac catcgagtgg tgacaaattc aagagctgca     840
   aggacaacga ttgcgtactt catttatccg tctaatgaga ccgttataga acctgcaaat     900
   gttttgtgca atccaccact gtacagatcc atgaagttta cggagttcct tcaacacttc     960
   aaatcgaaag ctgccaacga cgaagaaatg tcgaaggttt taagcctctc gagtagctaa    1020
<210> 2
   <211> 339
   <212> PRT
   <213> Malus x domestica Borkh
<400> 2
   Met Glu Thr Leu Asp Gln Asn Leu Val Ser Ser Trp Phe Asp Val Gln
   1               5                   10                  15
   Ser Val Pro Gln Thr Phe Val His Pro Pro Glu Lys Arg Pro Gly Asn
               20                  25                  30
   Ser Ile Asp Val Pro Pro Cys Lys Asn Ile Pro Val Val Asp Leu Gly
           35                  40                  45
   Ser Arg Asp Arg Ser His Thr Ile Gln Gln Ile Ser Lys Ala Ser Gln
       50                  55                  60
   Asp Phe Gly Phe Phe Gln Val Phe Asn His Gly Val Cys Lys Lys Leu
   65                  70                  75                  80
   Ile Asp Asp Ile Met Ser Ile Ser Lys Glu Phe His Lys Met Pro Arg
                   85                  90                  95
   Lys Asp Lys Ile Ile Glu Gly Ser Lys Asp Pro Ser Gly Arg Cys Lys
               100                 105                 110
   Phe Tyr Thr Ser Ser Glu Asn Tyr Ala Asn Glu Glu Val His Tyr Trp
           115                 120                 125
   Arg Asp Ala Leu Thr His Pro Ala His Ser Ser Glu Asn Tyr Met Gln
       130                 135                 140
   Phe Leu Pro Gln Lys Pro Thr Gln Tyr Arg Glu Val Phe Lys Ala Tyr
   145                 150                 155                 160
   Val Asp Glu Val Arg Asn Met Gly Ser Met Ile Leu Glu Met Leu Ala
                   165                 170                 175
   Glu Gly Leu Gly Leu Ser Lys Glu Phe Phe Asn Gly Gly Leu Ser Glu
               180                 185                 190
   Asn Pro Thr Leu Leu Ser Asn His Tyr Pro Pro Cys Pro Asp Pro Thr
           195                 200                 205
   Leu Thr Leu Gly Leu Thr Lys His Arg Asp Pro Ser Leu Ile Thr Ile
       210                 215                 220
   Leu Leu Gln Asp Ser Glu Gly Leu Gln Val Phe Lys Asp Gly Asn Trp
   225                 230                 235                 240
   Ile Gly Val Glu Pro Ile Ser Ser Gly Phe Val Val Asn Ile Gly Tyr
                   245                 250                 255
   Val Met Gln Met Ile Ser Asn Ser Lys Phe Lys Gly Ala Asp His Arg
               260                 265                 270
   Val Val Thr Asn Ser Arg Ala Ala Arg Thr Thr Ile Ala Tyr Phe Ile
           275                 280                 285
   Tyr Pro Ser Asn Glu Thr Val Ile Glu Pro Ala Asn Val Leu Cys Asn
       290                 295                 300
   Pro Pro Leu Tyr Arg Ser Met Lys Phe Thr Glu Phe Leu Gln His Phe
   305                 310                 315                 320
   Lys Ser Lys Ala Ala Asn Asp Glu Glu Met Ser Lys Val Leu Ser Leu
                   325                 330                 335
   Ser Ser Ser
<210> 3
   <211> 20
   <212> DNA
   <213> Artificial sequence
<220>
   <223> JseqSTMC1f Primer
<400> 3
   ccgaccacag ctacgaaaat 20
<210> 4
   <211> 26
   <212> DNA
   <213> Artificial sequence
<220>
   <223> JseqSTMC2r Primer
<400> 4
   aaaaactaaa aacgtttaac caaaca 26
<210> 5
   <211> 25
   <212> DNA
   <213> Artificial sequence
<220>
   <223> JseqSTMC3f Primer
<400> 5
   aaagaacatg gaagttgata attgg 25
<210> 6
   <211> 21
   <212> DNA
   <213> Artificial sequence
<220>
   <223> JseqSTMC5f Primer
<400> 6
   aaaggcactg cataggaaga a 21
<210> 7
   <211> 21
   <212> DNA
   <213> Artificial sequence
<220>
   <223> JseqSTMC6f Primer
<400> 7
   cacacttaag ggggaatgtt g 21
<210> 8
   <211> 20
   <212> DNA
   <213> Artificial sequence
<220>
   <223> JseqSTMC6r Primer
<400> 8
   ggggacactc tcttgatcgt 20
<210> 9
   <211> 26
   <212> DNA
   <213> Artificial sequence
<220>
   <223> JseqSTMC7r primer
<400> 9
   cagaaggatt aatgtcacaa caattt 26
<210> 10
   <211> 24
   <212> DNA
   <213> Artificial sequence
<220>
   <223> JseqWiC1f primer
<400> 10
   tcattctttt tgctacttcg ttga 24
<210> 11
   <211> 20
   <212> DNA
   <213> Artificial sequence
<220>
   <223> JseqWiC2f Primer
<400> 11
   agctcgggga tcatataggc 20
<210> 12
   <211> 23
   <212> DNA
   <213> Artificial sequence
<220>
   <223> JseqWiC2r Primer
<400> 12
   gtcccttaac tttgtacacc tca 23
<210> 13
   <211> 25
   <212> DNA
   <213> Artificial sequence
<220>
   <223> JseqWiC3f Primer
<400> 13
   aaaccttgta atttcatgca ttttt 25
<210> 14
   <211> 22
   <212> DNA
   <213> Artificial sequence
<220>
   <223> JseqWiC4f Primer
<400> 14
   gtacctggag cttttcgtta aa 22
<210> 15
   <211> 20
   <212> DNA
   <213> Artificial sequence
<220>
   <223> JseqWiC5f Primer
<400> 15
   tgcccaaaat acccctattg 20
<210> 16
   <211> 27
   <212> DNA
   <213> Artificial sequence
<220>
   <223> JseqWiC6r Primer
<400> 16
   tcttcttgtt atgggtaaat tattctc 27
<210> 17
   <211> 20
   <212> DNA
   <213> Artificial sequence
<220>
   <223> JseqWiC7f2 Primer
<400> 17
   tggttcatcc aaaccttcaa 20
<210> 18
   <211> 20
   <212> DNA
   <213> Artificial sequence
<220>
   <223> JseqWiC8r2 Primer
<400> 18
   cttccaagtc ttcggtcgag 20
<210> 19
   <211> 18
   <212> DNA
   <213> Artificial sequence
<220>
   <223> JseqWiC9r Primer
<400> 19
   taggccaaac tgcgtcct 18
<210> 20
   <211> 23
   <212> DNA
   <213> Artificial sequence
<220>
   <223> JseqWiC10f Primer
<400> 20
   aaaaccattc cttcaaaagt gat 23
<210> 21
   <211> 20
   <212> DNA
   <213> Artificial sequence
<220>
   <223> JseqWiC10r Primer
<400> 21
   gttactcagg ggtggctctg 20
<210> 22
   <211> 20
   <212> DNA
   <213> Artificial sequence
<220>
   <223> JseqWiC12f Primer
<400> 22
   caatttgctc tagcccatgt 20
<210> 23
   <211> 20
   <212> DNA
   <213> Artificial sequence
<220>
   <223> JseqWiC14f Primer
<400> 23
   agcccaaaac tcctcattca 20
<210> 24
   <211> 20
   <212> DNA
   <213> Artificial sequence
<220>
   <223> JseqWiC15f Primer
<400> 24
   cgccgtaaga gggaatagac 20
<210> 25
   <211> 20
   <212> DNA
   <213> Artificial sequence
<220>
   <223> JseqWiC16f Primer
<400> 25
   ggaaaacagg atggccacta 20
<210> 26
   <211> 24
   <212> DNA
   <213> Artificial sequence
<220>
   <223> JseqWiC17f Primer
<400> 26
   catgattcac aatatggttt ttga 24
<210> 27
   <211> 26
   <212> DNA
   <213> Artificial sequence
<220>
   <223> JseqWiC18f Primer
<400> 27
   gcaaataatt tacgtacact ttgaat 26
<210> 28
   <211> 20
   <212> DNA
   <213> Artificial sequence
<220>
   <223> JseqWiC19f2 Primer
<400> 28
   cggagatggt gtgcctattc 20
<210> 29
   <211> 20
   <212> DNA
   <213> Artificial sequence
<220>
   <223> JseqWiC19r Primer
<400> 29
   ttactccagc ggcaacttct 20
<210> 30
   <211> 22
   <212> DNA
   <213> Artificial sequence
<220>
   <223> JseqWiC20f Primer
<400> 30
   ctcgtctcca atcaaattgt gt 22
<210> 31
   <211> 21
   <212> DNA
   <213> Artificial sequence
<220>
   <223> JseqWiC20r Primer
<400> 31
   gcatttccat atgcctaacc a 21
<210> 32
   <211> 24
   <212> DNA
   <213> Artificial sequence
<220>
   <223> JseqWiC22f Primer
<400> 32
   tcaaattatt ttgcctaatg agaa 24
<210> 33
   <211> 25
   <212> DNA
   <213> Artificial sequence
<220>
   <223> JseqWiC22r Primer
<400> 33
   aaatttagtt caaacatttc gcagt 25
<210> 34
   <211> 25
   <212> DNA
   <213> Artificial sequence
<220>
   <223> JseqWiC23f Primer
<400> 34
   ggcaagatag actttatgaa ccaaa 25
<210> 35
   <211> 21
   <212> DNA
   <213> Artificial sequence
<220>
   <223> JseqWiC24f Primer
<400> 35
   agacacctca tgtgggaaaa a 21
<210> 36
   <211> 22
   <212> DNA
   <213> Artificial sequence
<220>
   <223> JseqWiC24r Primer
<400> 36
   cctgcataac ctttcttcat ca 22
<210> 37
   <211> 20
   <212> DNA
   <213> Artificial sequence
<220>
   <223> JseqWiC25f Primer
<400> 37
   gacgaaaatt gactgcacga 20
<210> 38
   <211> 26
   <212> DNA
   <213> Artificial sequence
<220>
   <223> JseqWiC25f2 Primer
<400> 38
   ggattacgca atgtaatcat agttct 26
<210> 39
   <211> 20
   <212> DNA
   <213> Artificial sequence
<220>
   <223> JseqWiC26r Primer
<400> 39
   cttcactcac gcacgtccta 20
<210> 40
   <211> 23
   <212> DNA
   <213> Artificial sequence
<220>
   <223> JseqWiC27f Primer
<400> 40
   catctttagg gatgtgtttt acg 23
<210> 41
   <211> 23
   <212> DNA
   <213> Artificial sequence
<220>
   <223> JseqWiC28f Primer
<400> 41
   tcaacgttgc aaaatctatc aaa 23
<210> 42
   <211> 20
   <212> DNA
   <213> Artificial sequence
<220>
   <223> JseqWiC28r Primer
<400> 42
   gcatcctacg ttcaccttcg 20
<210> 43
   <211> 21
   <212> DNA
   <213> Artificial sequence
<220>
   <223> JseqWiC29f Primer
<400> 43
   cctggatcta aaagccccat a 21
<210> 44
   <211> 21
   <212> DNA
   <213> Artificial sequence
<220>
   <223> JseqWiC29f2 Primer
<400> 44
   gctagagggt tcttctccac a 21
<210> 45
   <211> 21
   <212> DNA
   <213> Artificial sequence
<220>
   <223> JseqWiC30f Primer
<400> 45
   aaaaaccacc ctcaaatttc c 21
<210> 46
   <211> 20
   <212> DNA
   <213> Artificial sequence
<220>
   <223> JseqWiC30r Primer
<400> 46
   gctttgagaa ggggtgagaa 20
<210> 47
   <211> 20
   <212> DNA
   <213> Artificial sequence
<220>
   <223> JseqWiC31f3 Primer
<400> 47
   caacaatgtg cctcgacact 20
<210> 48
   <211> 20
   <212> DNA
   <213> Artificial sequence
<220>
   <223> JseqWiC32f Primer
<400> 48
   caagattcga agaacgatgc 20
<210> 49
   <211> 22
   <212> DNA
   <213> Artificial sequence
<220>
   <223> JseqWiC32r2 Primer
<400> 49
   catcaccttt catcacattt ca 22
<210> 50
   <211> 23
   <212> DNA
   <213> Artificial sequence
<220>
   <223> JseqWiC33f Primer
<400> 50
   tttttcgtct cagagtcata cct 23
<210> 51
   <211> 27
   <212> DNA
   <213> Artificial sequence
<220>
   <223> JseqWiC33f2 Primer
<400> 51
   tggaagacat taaataaaag ggtaaaa 27
<210> 52
   <211> 26
   <212> DNA
   <213> Artificial sequence
<220>
   <223> JseqWiC33r Primer
<400> 52
   cataaatttt gaaggtctag ctacca 26
<210> 53
   <211> 20
   <212> DNA
   <213> Artificial sequence
<220>
   <223> JseqWiC34f Primer
<400> 53
   actgcatatt tgccacgtca 20
<210> 54
   <211> 20
   <212> DNA
   <213> Artificial sequence
<220>
   <223> JseqWiC34r3 Primer
<400> 54
   ttccctcggc tttttattga 20
<210> 55
   <211> 20
   <212> DNA
   <213> Artificial sequence
<220>
   <223> JseqWiC35f Primer
<400> 55
   ttggtttgaa catccccttt 20
<210> 56
   <211> 21
   <212> DNA
   <213> Artificial sequence
<220>
   <223> JseqWiGAPC1f Primer
<400> 56
   tggtcttttc cttggaggag t 21
<210> 57
   <211> 22
   <212> DNA
   <213> Artificial sequence
<220>
   <223> JseqWiGAPC1r Primer
<400> 57
   tgacagcctt gtttaaacgg ta 22
<210> 58
   <211> 20
   <212> DNA
   <213> Artificial sequence
<220>
   <223> JseqWiGAPC2f Primer
<400> 58
   agtggctgtg ctcagtgatg 20
<210> 59
   <211> 20
   <212> DNA
   <213> Artificial sequence
<220>
   <223> JseqwiGAPC2r Primer
<400> 59
   gcattggagg aggagggtaa 20
<210> 60
   <211> 19
   <212> DNA
   <213> Artificial sequence
<220>
   <223> Forward primer - gene MdCo27
<400> 60
   ctcctagagg cccgagatg 19
<210> 61
   <211> 20
   <212> DNA
   <213> Artificial sequence
<220>
   <223> Reverse primer - gene MdCo27
<400> 61
   tgccgtgagt ttcctattcc 20
<210> 62
   <211> 19
   <212> DNA
   <213> Artificial sequence
<220>
   <223> Forward primer - gene MdCo28
<400> 62
   actgtggagg tcggaaacc 19
<210> 63
   <211> 20
   <212> DNA
   <213> Artificial sequence
<220>
   <223> Reverse primer - gene MdCo28
<400> 63
   aaggctcgta aatgccacag 20
<210> 64
   <211> 20
   <212> DNA
   <213> Artificial sequence
<220>
   <223> Forward primer - gene MdCo29
<400> 64
   ggatggggaa gggattagag 20
<210> 65
   <211> 20
   <212> DNA
   <213> Artificial sequence
<220>
   <223> Reverse primer - gene MdCo29
<400> 65
   ggagactgag gaaggcacat 20
<210> 66
   <211> 20
   <212> DNA
   <213> Artificial sequence
<220>
   <223> Forward primer - gene MdCo30
<400> 66
   ccgtctgtgg aacgacactt 20
<210> 67
   <211> 20
   <212> DNA
   <213> Artificial sequence
<220>
   <223> Reverse primer - gene MdCo30
<400> 67
   atgcgtgtca ttttgtgtgc 20
<210> 68
   <211> 20
   <212> DNA
   <213> Artificial sequence
<220>
   <223> Forward primer - gene MdCo31
<400> 68
   cataaaatgc cccgaaaaga 20
<210> 69
   <211> 21
   <212> DNA
   <213> Artificial sequence
<220>
   <223> Reverse primer - gene MdCo31
<400> 69
   cagaagaatg agcagggtga g 21
<210> 70
   <211> 20
   <212> DNA
   <213> Artificial sequence
<220>
   <223> Forward primer - gene MdCo32
<400> 70
   ccccgaagag acaaggaaac 20
<210> 71
   <211> 20
   <212> DNA
   <213> Artificial sequence
<220>
   <223> Reverse primer - gene MdCo32
<400> 71
   ccagaatgac agaccccaat 20
<210> 72
   <211> 25
   <212> DNA
   <213> Artificial sequence
<220>
   <223> Forward primer - gene MdActin
<400> 72
   tgaccgaatg agcaaggaaa ttact 25
<210> 73
   <211> 24
   <212> DNA
   <213> Artificial sequence
<220>
   <223> Reverse primer - gene MdActin
<400> 73
   tactcagctt tggcaatcca catc 24
<210> 74
   <211> 20
   <212> DNA
   <213> Artificial sequence
<220>
   <223> Forward primer - gene Md_4592:1:a
<400> 74
   gtcgaaatgg tcagcggtag 20
<210> 75
   <211> 20
   <212> DNA
   <213> Artificial sequence
<220>
   <223> Reverse primer - gene Md_4592:1:a
<400> 75
   gcaatggcaa actccacctt 20
<210> 76
   <211> 21
   <212> DNA
   <213> Artificial sequence
<220>
   <223> Forward Primer - SSR marker Co04R09
<400> 76
   tagtgacata tacatggtgc g 21
<210> 77
   <211> 20
   <212> DNA
   <213> Artificial sequence
<220>
   <223> Reverse Primer - SSR marker Co04R09
<400> 77
   gttggagaat gagtgacggc 20
<210> 78
   <211> 20
   <212> DNA
   <213> Artificial sequence
<220>
   <223> Forward Primer - SSR marker Co04R10
<400> 78
   acctggttcc ggtacatagc 20
<210> 79
   <211> 20
   <212> DNA
   <213> Artificial sequence
<220>
   <223> Reverse Primer - SSR marker Co04R10
<400> 79
   aaccttccat ggcagcaatc 20
<210> 80
   <211> 22
   <212> DNA
   <213> Artificial sequence
<220>
   <223> Forward Primer - SSR marker Co04R11
<400> 80
   acatcatggt atgacagagg tg 22
<210> 81
   <211> 20
   <212> DNA
   <213> Artificial sequence
<220>
   <223> Reverse Primer - SSR marker Co04R11
<400> 81
   tctaagcctg tcaagatggc 20
<210> 82
   <211> 21
   <212> DNA
   <213> Artificial sequence
<220>
   <223> Forward Primer - SSR marker J8Co
<400> 82
   tcgttgctca attactggct a 21
<210> 83
   <211> 20
   <212> DNA
   <213> Artificial sequence
<220>
   <223> Reverse Primer - SSR marker J8Co
<400> 83
   aggaatgcac aaatcctcca 20
<210> 84
   <211> 21
   <212> DNA
   <213> Artificial sequence
<220>
   <223> Forward Primer - SSR marker J9co
<400> 84
   tgttgaggga tttttcagac g 21
<210> 85
   <211> 20
   <212> DNA
   <213> Artificial sequence
<220>
   <223> Rverse Primer - SSR marker J9co
<400> 85
   gcactcccac cccactacta 20
<210> 86
   <211> 20
   <212> DNA
   <213> Artificial sequence
<220>
   <223> Forward Primer - SSR marker J11Co
<400> 86
   acccgagacc taccgcttat 20
<210> 87
   <211> 20
   <212> DNA
   <213> Artificial sequence
<220>
   <223> Reverse Primer - SSR marker J11Co
<400> 87
   tttgaatgtg gtccctagcc 20
<210> 88
   <211> 21
   <212> DNA
   <213> Artificial sequence
<220>
   <223> Forward Primer - SSR marker Co04R12
<400> 88
   tttatctgac taaggggaag g 21
<210> 89
   <211> 21
   <212> DNA
   <213> Artificial sequence
<220>
   <223> Reverse Primer - SSR marker Co04R12
<400> 89
   atggacttgt attccttagg g 21
<210> 90
   <211> 19
   <212> DNA
   <213> Artificial sequence
<220>
   <223> Forward Primer - SSR marker J12Co
<400> 90
   gcacccccag atttcaact 19
<210> 91
   <211> 20
   <212> DNA
   <213> Artificial sequence
<220>
   <223> Reverse Primer - SSR marker J12Co
<400> 91
   gctactgctg atggcctctc 20
<210> 92
   <211> 26
   <212> DNA
   <213> Artificial sequence
<220>
   <223> Forward Primer - SSR marker J14Co
<400> 92
   ctgaacataa caccctatac tttgaa 26
<210> 93
   <211> 24
   <212> DNA
   <213> Artificial sequence
<220>
   <223> Reverse Primer - SSR marker J14Co
<400> 93
   gacttcaaac ctgaagggat aaaa 24
<210> 94
   <211> 21
   <212> DNA
   <213> Artificial sequence
<220>
   <223> Forward Primer - SSR marker Co04R13
<400> 94
   attttccctc tcttctgttg c 21
<210> 95
   <211> 20
   <212> DNA
   <213> Artificial sequence
<220>
   <223> Reverse Primer - SSR marker Co04R13
<400> 95
   tcttggaaag acgtggcacg 20
<210> 96
   <211> 22
   <212> DNA
   <213> Artificial sequence
<220>
   <223> JseqSTMG1f primer
<400> 96
   aactgtcagg aatcggaata gg 22
<210> 97
   <211> 22
   <212> DNA
   <213> Artificial sequence
<220>
   <223> JseqSTMG1r primer
<400> 97
   tcaacatcat tcttgccatc tt 22
<210> 98
   <211> 21
   <212> DNA
   <213> Artificial sequence
<220>
   <223> JASSTMG2f
<400> 98
   tgtaaacgtg ctttcagcaa a 21
<210> 99
   <211> 20
   <212> DNA
   <213> Artificial sequence
<220>
   <223> JASSTMg1r primer
<400> 99
   agaagagttc cggtgagcaa 20
<210> 100
   <211> 22
   <212> DNA
   <213> Artificial sequence
<220>
   <223> JseqSTMG2f primer
<400> 100
   cacgtgtcaa tgtcagaatg aa 22
<210> 101
   <211> 22
   <212> DNA
   <213> Artificial sequence
<220>
   <223> JseqSTMG2r primer
<400> 101
   cgtccacaga gtagagttct ca 22
<210> 102
   <211> 20
   <212> DNA
   <213> Artificial sequence
<220>
   <223> JASSTMg3f primer
<400> 102
   tcaggaaaca atggggttct 20
<210> 103
   <211> 20
   <212> DNA
   <213> Artificial sequence
<220>
   <223> JASSTMg2r
<400> 103
   tggtgaaaat gatggggttt 20
<210> 104
   <211> 20
   <212> DNA
   <213> Artificial sequence
<220>
   <223> JseqSTMg3f primer
<400> 104
   ttggcccaaa atccaaataa 20
<210> 105
   <211> 22
   <212> DNA
   <213> Artificial sequence
<220>
   <223> JseqSTMg3r primer
<400> 105
   cttcaagttt tcatgggtga tg 22
<210> 106
   <211> 20
   <212> DNA
   <213> Artificial sequence
<220>
   <223> JASSTMg4f primer
<400> 106
   tccattggag gaaacaaagc 20
<210> 107
   <211> 20
   <212> DNA
   <213> Artificial sequence
<220>
   <223> JASSTMg3r primer
<400> 107
   ttgtggtctc cagttcaacg 20
<210> 108
   <211> 20
   <212> DNA
   <213> Artificial sequence
<220>
   <223> JseqSTMg4f primer
<400> 108
   tgcatcgaat cctcaaatca 20
<210> 109
   <211> 20
   <212> DNA
   <213> Artificial sequence
<220>
   <223> JseqSTMg4r primer
<400> 109
   atggtccgta tggaaggtga 20
<210> 110
   <211> 20
   <212> DNA
   <213> Artificial sequence
<220>
   <223> JASSTMg5f primer
<400> 110
   ggcgtcaata ttggtgatcc 20
<210> 111
   <211> 27
   <212> DNA
   <213> Artificial sequence
<220>
   <223> JASSTMg4r primer
<400> 111
   aaactagcat aatagatcga aatctca 27
<210> 112
   <211> 20
   <212> DNA
   <213> Artificial sequence
<220>
   <223> JSeqsTMg5f primer
<400> 112
   tgctctcaat tcgactgtgg 20
<210> 113
   <211> 20
   <212> DNA
   <213> Artificial sequence
<220>
   <223> JSeqsTMg5r primer
<400> 113
   gttctcgcga tctcttcagg 20
<210> 114
   <211> 20
   <212> DNA
   <213> Artificial sequence
<220>
   <223> JASSTMg6f primer
<400> 114
   acgcatcatg catcaaacag 20
<210> 115
   <211> 20
   <212> DNA
   <213> Artificial sequence
<220>
   <223> JASSTMg5r primer
<400> 115
   ccaatatggg tgcctcgtta 20
<210> 116
   <211> 20
   <212> DNA
   <213> Artificial sequence
<220>
   <223> JseqSTMg6f primer
<400> 116
   gacttgctgc ttcgtgcata 20
<210> 117
   <211> 20
   <212> DNA
   <213> Artificial sequence
<220>
   <223> JseqSTMg6f2 primer
<400> 117
   acgtccacgg aatagaggag 20
<210> 118
   <211> 22
   <212> DNA
   <213> Artificial sequence
<220>
   <223> JseqSTMg6r primer
<400> 118
   ttttaaaccc aagacctcct ca 22
<210> 119
   <211> 24
   <212> DNA
   <213> Artificial sequence
<220>
   <223> JASSTMg7f primer
<400> 119
   gactcaacaa caacaacaac aaca 24
<210> 120
   <211> 20
   <212> DNA
   <213> Artificial sequence
<220>
   <223> JASSTMg6r primer
<400> 120
   ggaagtcaag ccgataccac 20
<210> 121
   <211> 20
   <212> DNA
   <213> Artificial sequence
<220>
   <223> JSeqsTMg7f primer
<400> 121
   aggccgactg aagaatttga 20
<210> 122
   <211> 20
   <212> DNA
   <213> Artificial sequence
<220>
   <223> JSeqsTMg7r primer
<400> 122
   cttaccctgg cattgctctc 20
<210> 123
   <211> 27
   <212> DNA
   <213> Artificial sequence
<220>
   <223> JASSTMg7r2 primer
<400> 123
   tctaacaaag ttgagactac ttttcca 27
<210> 124
   <211> 21
   <212> DNA
   <213> Artificial sequence
<220>
   <223> 29f1 primer
<400> 124
   cctggatcta aaagccccat a 21
<210> 125
   <211> 20
   <212> DNA
   <213> Artificial sequence
<220>
   <223> JWI1r primer
<400> 125
   aaccaaacac ccacccatta 20
<210> 126
   <211> 21
   <212> DNA
   <213> Artificial sequence
<220>
   <223> 29f2 primer
<400> 126
   gctagagggt tcttctccac a 21
<210> 127
   <211> 20
   <212> DNA
   <213> artificial sequence
<220>
   <223> JWI3r primer
<400> 127
   gtaggagagt ccggggaaag 20
<210> 128
   <211> 24
   <212> DNA
   <213> Artificial sequence
<220>
   <223> 29f3 primer
<400> 128
   tgatctacac agacagtttt gacg 24
<210> 129
   <211> 20
   <212> DNA
   <213> Artificial sequence
<220>
   <223> 30r primer
<400> 129
   gctttgagaa ggggtgagaa 20

## Description

### Technical Field

The present invention falls within the section of human necessities, in the field of agriculture; new plants or processes for obtaining them by means of biotechnology and genetic engineering; ornamental and fruit plants, and especially apple trees. In particular, the present invention relates to a Co gene of the 'Wijcik' mutant of *Malus x domestica Borkh*, and other plants genetically transformed by introduction of this gene, wherein these plants have shorter axillary shoots than a control plant.

### State of the Art

Tree architecture is one of the most important traits that a breeder must consider when selecting a new improved variety in all woody plants. Such trait is particularly important in fruit trees, as it can influence most of the parameters of an orchard management, such as plant density, pruning, training, the need and the costs of a support system and so on. More over tree architecture deeply influences a lot of relevant characteristics of the plant, such as fruit yield and quality, canopy light capture and balance between vegetative and reproductive shoots. Tree architecture is a very complex trait and can be influenced by a lot of environmental factors such as temperature, water availability and fertilization. Nevertheless the genetic component is relevant and it is known since a long time that each tree presents a precisely determined genetic growth plan. The traits involved are numerous and include such factors as whether the tree branches or remains unbranched (as in palms for example), whether or not branching is restricted to the top of the plant or extended till the base of the trunk, if the growth of the branches will be continuous (as in tropical species) or rhythmic, the type and number of branches that can be very short (spurs) or long and slender and finally even the insertion angle of the branches to the trunk. Therefore it is easy to understand that it is very difficult to control all the parameters that in the end will determine plant architecture. In general we can say that for the modern concept of orchard, plants should be quite compact in order to allow high density planting and reduce considerably the need of pruning that is the common practice to reduce the tree crown. As a matter of fact pruning is not only a costly practice (in terms of time and money) but presents other disadvantages because severe pruning actually invigorates trees and induces excessive vegetative growth and in turn a decrease of fruit production. Most of the rootstocks used for fruit trees growing are selected in order to reduce plant vigour.

In apple (*Malus x domestica Borkh),* tree architecture has a direct influence on plant vigour, balance between vegetative and reproductive shoots, canopy light capture, and influences considerably yield and quality of fruits (Lespinasse, 1993; Wünsche, 2000; Barritt, 1992). Moreover, orchard management, including pruning, training, as well as plant density and tree support systems are directly influenced by tree architecture (Miller, 2001; Thorp, 2001).

The apple columnar mutant 'Wijcik" was first identified in the 60's as a shoot on top of a normal 'McIntosh' tree (Fisher, 1969). 'Wijcik' trees have short internodes and a thick stem. Axillary buds develop into spurs rather than lateral branches, resulting in a compact, columnar tree, with fruits produced close to the stem (Fisher, 1969; Tobutt, 1985). Columnar growth could be very beneficial for apple production as it greatly reduces the need for pruning, resulting in a decrease of labor required for maintaining trees. Furthermore, columnar trees allow for high density planting, leading to yield increase (Tobutt, 1985). The columnar phenotype was shown to be caused by a single, dominant allele (Co), mapping on linkage group 10 (Conner, 1997). Several molecular markers have been developed for Co, but till recently they were too distant from each other to allow the identification of the actual gene responsible for the columnar phenotype (Conner, 1997; Hemmat, 1997; Kim, 2003; Tian, 2005; Zhu, 2007; Moriya, 2009).

In 2012, Baldi et al. identified a candidate region delimited by SSR markers Co04R10 and Co04R13, with a genetic distance of 0,56 cM. from each other. The physical distance between these markers in the homologous region of *Golden Delicious* was shown to be 393 kb and contains the region that was reported as candidate in a second work by Moriya et al. (2012). The findings of Bai et al. (2012) are in disagreement with these works since they reported a region for the Co gene with an estimated size of 193 kb located just outside the region described by Baldi et al. (2012) and Moriya et al. (2012).

Two transcriptome studies were also recently carried out in order to better understand the mechanism that leads to columnar growth (Krost, 2012; Zhang, 2012). Both studies reported several candidates as the potential Co gene and 4 emphasized transcription factors and enzymes involved in plant hormone signaling and particularly DELLA proteins involved in the regulation of the gibberellin pathway (Zhang, 2012). Mutations in DELLA proteins were shown to be the basis for the 'Green Revolution' that changed the way of cultivating maize, rice and wheat, allowing the development of more compact new cultivars with phenotypes similar to that of the 'Wijcik' mutant, (Peng, 1999).

Despite considerable efforts, the Co gene which leads to columnar growth in apple trees could not yet be isolated and there are still missing methods to specifically transform plants with normal branch growth into plants with controlled columnar growth. The known commercially available columnar apple trees are classic breeding results obtained from lengthy crossing with respective columnar mutants. Even though it has been known for a long time that the columnar growth of apple trees is genetically motivated, to this day a targeted use of this genetic information to create plants with columnar growth has not been described.

### Disclosure of the Invention

Scope of the present invention is to produce plants that have effectively a controlled tree architecture, in particular a substantial reduction in the length of branches in order to obtain plants with better fruits and also to improve the mechanical harvesting of these fruits.

A further scope of the invention is to provide plants which have the advantage to facilitate the pruning and need less pruning avoiding infections of the plant. Yet another scope of the invention is to modify apple trees, and plants in general, by genetic engineering to induce columnar growth.

The present invention is susceptible of industrial application, namely for avoid the deviation of assimilates away from developing fruits and also improve the mechanical harvesting of fruits.

These scopes and others, which are described in the following in more detail, are obtained by an isolated polypeptide according to the first claim. The name attributed to the polynucleotide according to the invention, thus the Co gene, is MdCo31.

The term "% identical" as is understood in this invention refers to the % of identity between two amino acid or nucleotide sequences. The % of identity is a count of the number of positions over the length of the alignment of two sequences where all of the amino acids/nucleotides at that position are identical. The methods to compare the sequences obtaining identity percentages are known in the state of the art, and are not described in detail.

In the present study, a direct sequence comparison between the Co region of 'Wijcik' and its wild-type cultivar 'McIntosh' was performed using two BAC libraries. Based on the sequence comparison, an insertion of 1956 bp was identified in the 'Wijcik' genome. The expression of genes located on the Co region was compared between 'McIntosh' and 'Wijcik' and resulted in the identification of a 2OG-Fe(II) oxygenase-like gene as being the gene responsible for the columnar phenotype.

Preferably, the polypeptide has the amino acid sequence of SEQ ID NO: 2. In one embodiment of the present invention, the nucleotide sequence encoding the polypeptide has at least 90%, preferably at least 95%, more preferably at least 98% sequence identity to the nucleotide sequence of SEQ ID NO: 1. Preferably the polynucleotide has the nucleotide sequence of SEQ ID NO: 1.

The polynucleotide according to the invention, a Co gene, encodes 2OG-Fe(II) oxygenase-like gene.

A preferred embodiment of the present invention refers to the polynucleotide operatively linked to any regulatory sequence that controls the expression of said polynucleotide, thus to a gene construct. The term "regulatory sequence that controls the expression of a polynucleotide" refers to a nucleotidic sequence having an effect on the functionality of the polynucleotide gene referred to the beginning of the transcript from a DNA sequence or at the start of translation of a sequence of RNA or other sequences not described. By way of example, between regulatory sequences of the gene expression referred to in the present invention are the promoters and other less common as certain introns. The nature of these regulatory sequences differs depending on the host organism; in prokaryotes, these sequences of control usually include a promoter, a site of union ribosomal, and signs of completion; in eukaryotes, usually, these sequences of control include promoters, signs of termination, intensifiers and sometimes silencers. As it is used here, the term "promoter" refers to a region of the DNA upstream from the start point of the transcription of a gene, and particularly, that is able to start the transcription in a plant cell. Examples of promoters include but are not limited to, promoters obtained from plants, virus of plants or bacteria that can express genes in cells of plants. Promoters can be classified, for instance, as inducible and constitutive promoters.

The invention further concerns an expression product of the polynucleotide according to the invention and a vector comprising the polynucleotide according to the invention. The term "expression product of the polynucleotide" as it is used in the present invention refers to any product derived from the expression of the polynucleotide of the invention. Thus, as product derived from the expression of the polynucleotide of the invention is understood, for example, the RNA that is obtained from the transcription of said polynucleotide, the processed RNA, the resulting protein in the translation of RNA, or subsequent modifications to the polynucleotide inside the cell, provided that the resulting sequence of these modified polynucleotides having its origin in the original sequence or the functional role described in the present invention.

The term "vector" refers to a DNA fragment that has the ability to replicate in a given host and, as the term indicates, it can serve as a vehicle to multiply another fragment of DNA that has being linked to the same (insert). The term "insert" refers to a DNA fragment that is linked to the vector; in the case of the present invention, the vector comprises any of the polynucleotides of the invention that can be replicated in the appropriate host. The vectors may be plasmids, cosmids, bacteriophages or viral vectors, without excluding another type of vectors that correspond to this definition of vector.

A further aspect of the invention concerns a host cell transformed with the polynucleotide according to the invention to express the polypeptide as defined in the polynucleotide claims. Another aspect of the present invention refers to a host cell comprising the polynucleotide, the expression product, the gene construct or the vector of the invention, wherein if the host cell is a plant cell, said plant cell is not from the mutant 'Wijcik'. A preferred embodiment refers to the host cell of the present invention, wherein said cell is a plant cell. More preferably the plant cell is a *Malus x domestica Borkh* cell. In another preferred variant the plant cell is not an apple tree cell, thus the polynucleotide according to the present invention is present in said host cell in heterologous form. The term "host cell" as it is used in the present invention relates to any prokaryotic or eukaryotic cell, essentially, it refers to an eukaryotic plant cell wherein any of these cells comprises the polynucleotide, the expression product, the gene construct or the vector of the invention. Thus, the term plant cell comprises at least one cell of the parenchyma, meristematic cell or of any kind of plant cell, differentiated or undifferentiated.

The present invention further relates to a cell culture comprising the host cell of the invention. The term "cell culture" refers to a cultivation of cells isolated from the same or different type of tissue, or a collection of these cells organized in parts of a plant or in tissues (tissue culture). Types of this kind of cultures are, e.g. but without any limitation, a culture of protoplasts, calli (groups of plant cells undifferentiated able to regenerate a complete plant with the appropriate organogenic program) or a culture of plant cells that are isolated from plants or parts of plants such as embryos, meristematic cells, pollen, leaves or anthers.

Another aspect of the present invention refers to a recombinant expression system comprising the polynucleotide, the expression product, the vector, the gene construct or the host cell of the invention. The recombinant expression system can be, but without limitation, e.g. a recombinant host cell or a recombinant bacteriophage or their combination with any helper virus. Recombinant manufacturing involves the expression of a DNA construct encoding for the desired protein in a recombinant host cell. The host cell can be either prokaryotic or eukaryotic. Expression constructs must be optimized for a particular protein and for a particular host cell. Expressing a recombinant protein in a host cell exposes the recombinant protein to a new set of host cell enzymes, such as proteases, which can modify or even degrade the recombinant protein. Modification and degradation of the recombinant protein of interest is undesirable.

An important aspect of the invention is a plant cell or plant transformed with the polynucleotide according to the invention to express the relative polypeptide, and wherein expression of said polypeptide results in reduced branch and/or trunk growth, preferably in shorter axillary shoots in said transformed plant cell or plant. The plant contains the polynucleotide of the invention in homozygosis, heterozygosis or hemicigosis. A further embodiment of the present invention refers to any plant described above, wherein the polynucleotide is integrated in their genome, preferably integrated in homozygosis.

Another aspect of the present invention refers to a plant, but not a *Wijcik* mutant plant, comprising the polynucleotide, the expression product, the gene construct, the vector, the host cell, the cell culture, or the recombinant expression system of the invention, wherein said plant has shorter axillary shoots than a control plant. Preferably the plant belongs to the species *Malus x domestica Borkh.* In another preferred embodiment of the invention, the plant is no apple tree.

In a preferred embodiment of the present invention, the plant cell or plant is not of the 'Wijcik' mutant wherein the plant has shorter axillary shoots than a non transformed control plant.

The control plant does not contain the polynucleotide of the present invention. The control plant has the same taxonomic category than the plant comprising the polynucleotide of the invention in other to compare the length of lateral branches.

Another advantageous aspect of the invention is a plant, preferably an apple tree, comprising, as a result of genetic transformation a fragment of the chromosome 10 of the 'Wijcik' mutant of *Malus x domestica Borkh* that comprises a polynucleotide encoding a polypeptide which is at least 90%, preferably at least 95%, more preferably at least 98% identical to SEQ ID NO: 2 or which is SEQ ID NO: 2, wherein said plant has shorter axillary shoots than a control plant.

Preferably, in the plant cell or plant according to the invention the expression of the polynucleotide is greater than the expression of the respective homologous native Co gene. Advantageously, this overexpression results in an increased 2OG-Fe(II) oxygenase-like production.

Still another aspect of the present invention refers to a method for producing a plant cell or plant with controlled tree architecture, the method comprising the steps of transformation of a plant cell or plant with the polynucleotide according to the invention and expression of the polypeptide encoded by said polynucleotide in the transformed plant cell or plant, and wherein said expression of said polypeptide reduces branch and/or trunk growth in said transformed plant.

The genetically transformed plant of the invention can be achieved by any technique known by the skilled in the art that will allow the integration of the polynucleotide of the invention in any of the DNA of the plant.

To obtain the genetically modified plant according to the invention the use of genetic transformation is needed, that is the introduction in the plant of a given gene, here the MdCo31 gene, under the control of a promoter of choice. Nowadays we can distinguish between two types of genetic transformation, according to the type of gene and promoter that are introduced in the plant. If the introduced gene comes from the same species or from a naturally crossable species and it is under the control of its own promoter, then we speak of *"cisgenesis"*; in all other cases we speak of *"transgenesis"* (for review see also Jacobsen and Schouten 2008; Schouten et al. 2006).

Some experiment of plant transformation with MdCo31 in different plant species, in particular *Arabidopsis* and tomato, two herbaceous model species with short living cycle, are under investigation, further experiments are carried out with some woody species such as poplar and apple with promising results regarding the length-controlled growth of branches and trunk. The state of the art offers to this purpose a lot of promoters, starting from the more common constitutive ones, such as CaMV 35S (Frank et al. 1980), nos (Depicker et al. 1982) and ubiquitin promoters (Christensen et al. 1992). Other promising promoters include but are not limited to tissue-specific promoters such as extA (Elliot and Shirsat 1998), CoYMV (Bedberry et al. 1990), roIC (Hu et al. 2003) and RSs1 (Wang et al. 1992) or some inducible promoters, such as the recently identified snakin-1 (Almasia et al. 2010); for review see also Zuo and Chua 2000. Obviously, some apple-specific promoters can also be cloned or developed.

Still another aspect of the invention, concerns a use of the polynucleotide, of the vector, of the expression product, of the genetic construct or of the host cell according to the invention, to control branch and trunk growth in a plant, preferably in a plant selected from fruit trees, in particular apple trees, or ornamental trees wherein the controlled branch and trunk growth preferably results in a predefined length of axillary shoots.

By performing a BLAST search, over a hundred of sequences homologous to the 'Wijcik' insertion were found distributed on *Golden Delicious* reference genome. No description of a similar sequence could be found either in plant gene databases, or in genomes of closely related *Rosaceae* species, like strawberry and peach, indicating that the inserted DNA element in 'Wijcik' Co region is specific to apple and has evolved recently.

Nevertheless, the polynucleotide of the invention isolated from the 'Wijcik' mutant can be used to transform other plant species, not only apple tree species, conferring them columnar growth characteristics, which can further be influenced in their extent, namely the length of the branches, using respective promoters.

In the plants of the present invention, the length of the basal lateral branches presents significant differences with regard to the length of the lateral branches in the control plants. In the present invention, the term "shorter" relates to significant differences between the length of axillary shoots, respectively, in the equivalent node of the plants of the invention and control plants. The skilled person can arrive to these significant differences using any statistical and any method known in the background art. Then, in the present invention plants having shorter axillary shoots than a control plant have branchless phenotype. Finally, the invention regards a germ plasm of the plant according to the invention, preferably a seed or pollen, containing the polynucleotide of anyone of the claims 1) - 4).

The term "plant" includes each of the parties on said plant, which can be conserved or cultivated in isolation or in combination, as well as the germplasm. The germplasm is defined by the biological material that contains the intraspecific genetic variability or by the genetic materials that can perpetuate a species or a population of said plant. Thus germplasm is the seed, tissue culture for any part of the plant or plants established in *ex situ* collections, without excluding any other material in the scope of this definition.

The pollen has high level of interest since the transmission of the genetic and phenotypic characters can be carried out by the pollination of any plant variety compatible with the pollen that is referenced. In this way it can be produced a plant which includes the polynucleotide of the invention, after the respective cross and selection, it can be obtained a plant in which the polynucleotide integrates a suitable number of copies in stable condition in order to obtain the same desirable branchless phenotype in the subsequent generations.

The plant cell or plant according to the invention can be a fruit tree, for example an apple tree, an ornamental plant or any other plant. The method or use of the invention can be applied to the above mentioned plants.

Further embodiments of the invention result from the dependent claims.

As would be appreciated by a person skilled in the art, many variations are possible without departing from the scope of the invention.

### Description of preferred embodiments

- Figure 1: depicts the alignment of the genomic region of 'Wijcik' containing the insertion with the 25 closest matches from *Golden Delicious.*
- Figure 2: shows an overview of the positions of sequences homologous to the 'Wijcik' insertion in the *Golden Delicious* genome.
- Figure 3: depicts the BAC contig construction.
- Figures 4A and 4B: show the BAC sequence assembly and identification of 'Wijcik' insertion.
- Figures 5A and 5B: show the annotation of the insert region and qPCR results.
- Figures 6A and 6B: show the insert amplification and MdCo31 expression in standard and columnar progeny trees.

### Materials and Methods

Leaf material and axillary buds were collected and ground to a fine powder in liquid nitrogen. Genomic DNA was extracted from 100 mg of ground material using the DNeasy Plant Mini Kit (QIAGEN). Total RNA isolation was performed using the RNeasy Mini Kit (QIAGEN). The *Golden Delicious* genome was used to search for potential SSRs using IMEx software (Mudunuri, 2007). PCR amplification of SSRs was performed in a volume of 10 µl with 1 µl of DNA template, 0,2 mM dNTPs, 3 mM MgCl2, 0,5 units of AmpliTaq Gold DNA polymerase (Applied Biosystems) and 0,1 mM of each primer. The cycling conditions include an initial denaturation step of 10 min at 95°C followed by 35 cycles of 30 s at 95°C, 1 min at 60°C, 30 s at 72°C and a final extension phase of 10 min at 72°C. PCR products were detected using a 3730 DNA analyzer (Applied Biosystems). The sizes of the different PCR products were determined using an internal size standard (MassRuler Low Range DNA Ladder; Fermentas, Glen Burnie, MD).

The 'Wijcik' library was already reported in Baldi et al. (2012). A second library was prepared by Amplicon express (Pullman, WA, USA) from 'McIntosh' leaf material as described in Baldi et al. (2012).

DNA from all 384-wells BAC plates was pooled and isolated using an alkaline lysis protocol adapted for purification of 96 samples at a time (Baldi, 2012). The pooled plates were screened using SSR markers developed for the Co region. Row and column pools were prepared from the positive plates and screened in order to identify the positive clones. The PCR conditions were the same as described above for the SSR analysis. BAC plasmids were purified using the Plasmid Midi kit (QIAGEN) and sequenced on a GS FLX Titanium sequencer (454 Life Sciences). The reads were assembled using Newbler Assembler software (Roche), MIRA assembler (Chevreux, 1999) and the *Golden Delicious* genome as a reference.

In order to join the contigs resulting from the assembly of the 454-reads, primers were designed on the contig ends (see table 1) and used for Sanger sequencing.

**Table 1. Primers used to complete the 'McIntosh' and 'Wijcik' BAC assembly**

| **Primer name** | **Sequence (5'-3')** | **SEQ ID NO:** |
|---|---|---|
| JseqSTMC1f | CCGACCACAGCTACGAAAAT | SEQ ID NO: 3 |
| JseqSTMC2r | AAAAACTAAAAACGTTTAACCAAACA | SEQ ID NO: 4 |
| JseqSTMC3f | AAAGAACATGGAAGTTGATAATTGG | SEQ ID NO: 5 |
| JseqSTMC5f | AAAGGCACTGCATAGGAAGAA | SEQ ID NO: 6 |
| JseqSTMC6f | CACACTTAAGGGGGAATGTTG | SEQ ID NO: 7 |
| JseqSTMC6r | GGGGACACTCTCTTGATCGT | SEQ ID NO: 8 |
| JseqSTMC7r | CAGAAGGATTAATGTCACAACAATTT | SEQ ID NO: 9 |
| JseqWiC1f | TCATTCTTTTTGCTACTTCGTTGA | SEQ ID NO: 10 |
| JseqWiC2f | AGCTCGGGGATCATATAGGC | SEQ ID NO: 11 |
| JseqWiC2r | GTCCCTTAACTTTGTACACCTCA | SEQ ID NO: 12 |
| JseqWiC3f | AAACCTTGTAATTTCATGCATTTTT | SEQ ID NO: 13 |
| JseqWiC4f | GTACCTGGAGCTTTTCGTTAAA | SEQ ID NO: 14 |
| JseqWiC5f | TGCCCAAAATACCCCTATTG | SEQ ID NO: 15 |
| JseqWiC6r | TCTTCTTGTTATGGGTAAATTATTCTC | SEQ ID NO: 16 |
| JseqWiC7f2 | TGGTTCATCCAAACCTTCAA | SEQ ID NO: 17 |
| JseqWiC8r2 | CTTCCAAGTCTTCGGTCGAG | SEQ ID NO: 18 |
| JseqWiC9r | TAGGCCAAACTGCGTCCT | SEQ ID NO: 19 |
| JseqWiC10f | AAAACCATTCCTTCAAAAGTGAT | SEQ ID NO: 20 |
| JseqWiC10r | GTTACTCAGGGGTGGCTCTG | SEQ ID NO: 21 |
| JseqWiC12f | CAATTTGCTCTAGCCCATGT | SEQ ID NO: 22 |
| JseqWiC14f | AGCCCAAAACTCCTCATTCA | SEQ ID NO: 23 |
| JseqWiC15f | CGCCGTAAGAGGGAATAGAC | SEQ ID NO: 24 |
| JseqWiC16f | GGAAAACAGGATGGCCACTA | SEQ ID NO: 25 |
| JseqWiC17f | CATGATTCACAATATGGTTTTTGA | SEQ ID NO: 26 |
| JseqWiC18f | GCAAATAATTTACGTACACTTTGAAT | SEQ ID NO: 27 |
| JseqWiC19f2 | CGGAGATGGTGTGCCTATTC | SEQ ID NO: 28 |
| JseqWiC19r | TTACTCCAGCGGCAACTTCT | SEQ ID NO: 29 |
| JseqWiC20f | CTCGTCTCCAATCAAATTGTGT | SEQ ID NO: 30 |
| JseqWiC20r | GCATTTCCATATGCCTAACCA | SEQ ID NO: 31 |
| JseqWiC22f | TCAAATTATTTTGCCTAATGAGAA | SEQ ID NO: 32 |
| JseqWiC22r | AAATTTAGTTCAAACATTTCGCAGT | SEQ ID NO: 33 |
| JseqWiC23f | GGCAAGATAGACTTTATGAACCAAA | SEQ ID NO: 34 |
| JseqWiC24f | AGACACCTCATGTGGGAAAAA | SEQ ID NO: 35 |
| JseqWiC24r | CCTGCATAACCTTTCTTCATCA | SEQ ID NO: 36 |
| JseqWiC25f | GACGAAAATTGACTGCACGA | SEQ ID NO: 37 |
| JseqWiC25f2 | GGATTACGCAATGTAATCATAGTTCT | SEQ ID NO: 38 |
| JseqWiC26r | CTTCACTCACGCACGTCCTA | SEQ ID NO: 39 |
| JseqWiC27f | CATCTTTAGGGATGTGTTTTACG | SEQ ID NO: 40 |
| JseqWiC28f | TCAACGTTGCAAAATCTATCAAA | SEQ ID NO: 41 |
| JseqWiC28r | GCATCCTACGTTCACCTTCG | SEQ ID NO: 42 |
| JseqWiC29f | CCTGGATCTAAAAGCCCCATA | SEQ ID NO: 43 |
| JseqWiC29f2 | GCTAGAGGGTTCTTCTCCACA | SEQ ID NO: 44 |
| JseqWiC30f | AAAAACCACCCTCAAATTTCC | SEQ ID NO: 45 |
| JseqWiC30r | GCTTTGAGAAGGGGTGAGAA | SEQ ID NO: 46 |
| JseqWiC31f3 | CAACAATGTGCCTCGACACT | SEQ ID NO: 47 |
| JseqWiC32f | CAAGATTCGAAGAACGATGC | SEQ ID NO: 48 |
| JseqWiC32r2 | CATCACCTTTCATCACATTTCA | SEQ ID NO: 49 |
| JseqWiC33f | TTTTTCGTCTCAGAGTCATACCT | SEQ ID NO: 50 |
| JseqWiC33f2 | TGGAAGACATTAAATAAAAGGGTAAAA | SEQ ID NO: 51 |
| JseqWiC33r | CATAAATTTTGAAGGTCTAGCTACCA | SEQ ID NO: 52 |
| JseqWiC34f | ACTGCATATTTGCCACGTCA | SEQ ID NO: 53 |
| JseqWiC34r3 | TTCCCTCGGCTTTTTATTGA | SEQ ID NO: 54 |
| JseqWiC35f | TTGGTTTGAACATCCCCTTT | SEQ ID NO: 55 |
| JseqWiGAPC1f | TGGTCTTTTCCTTGGAGGAGT | SEQ ID NO: 56 |
| JseqWiGAPC1r | TGACAGCCTTGTTTAAACGGTA | SEQ ID NO: 57 |
| JseqWiGAPC2f | AGTGGCTGTGCTCAGTGATG | SEQ ID NO: 58 |
| JseqWiGAPC2r | GCATTGGAGGAGGAGGGTAA | SEQ ID NO: 59 |

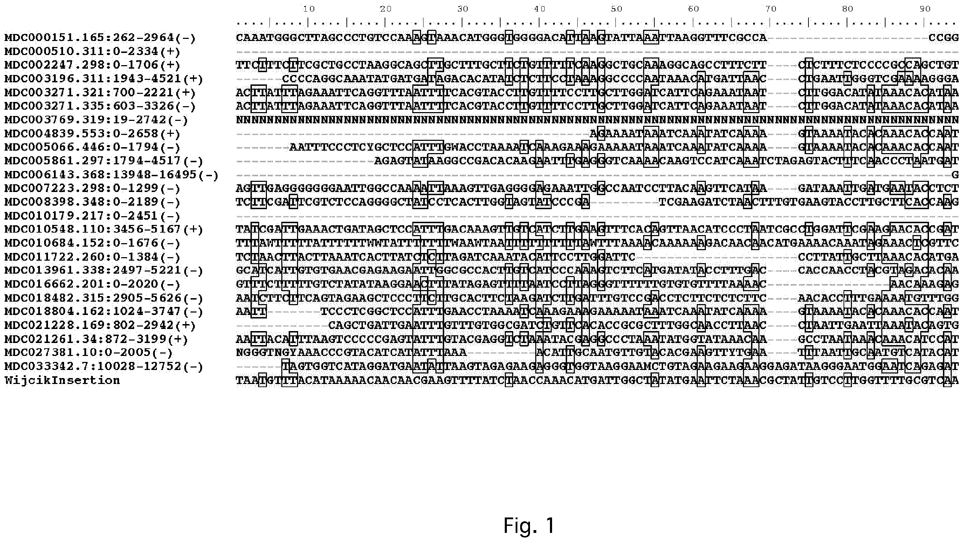

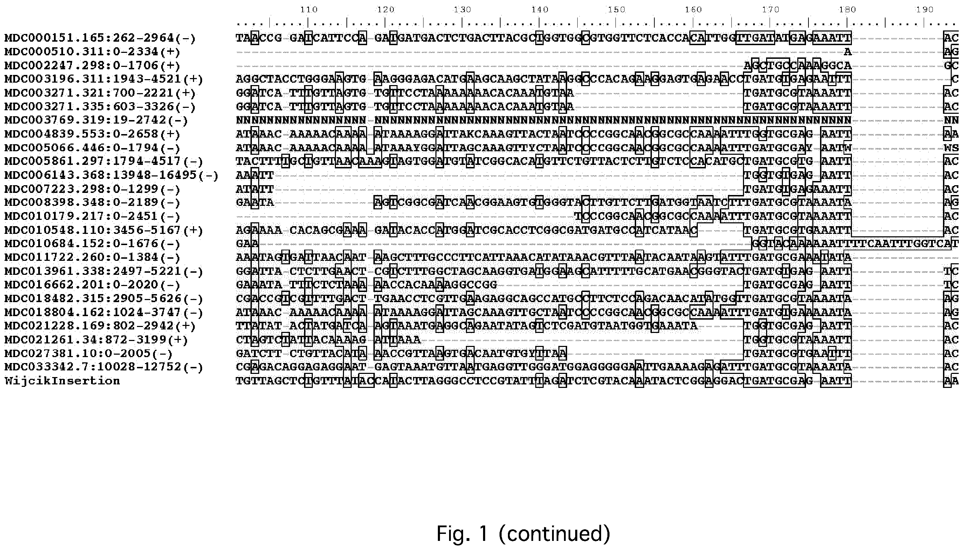

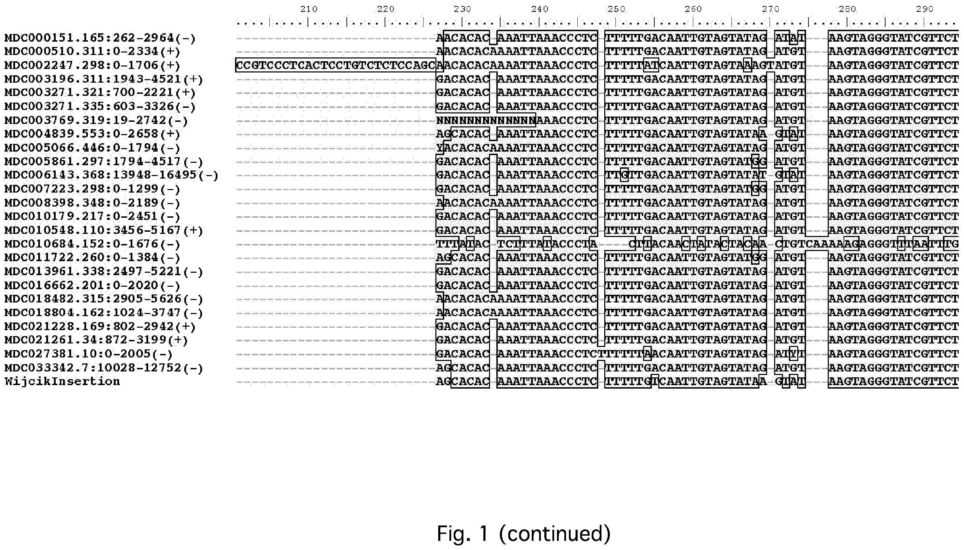

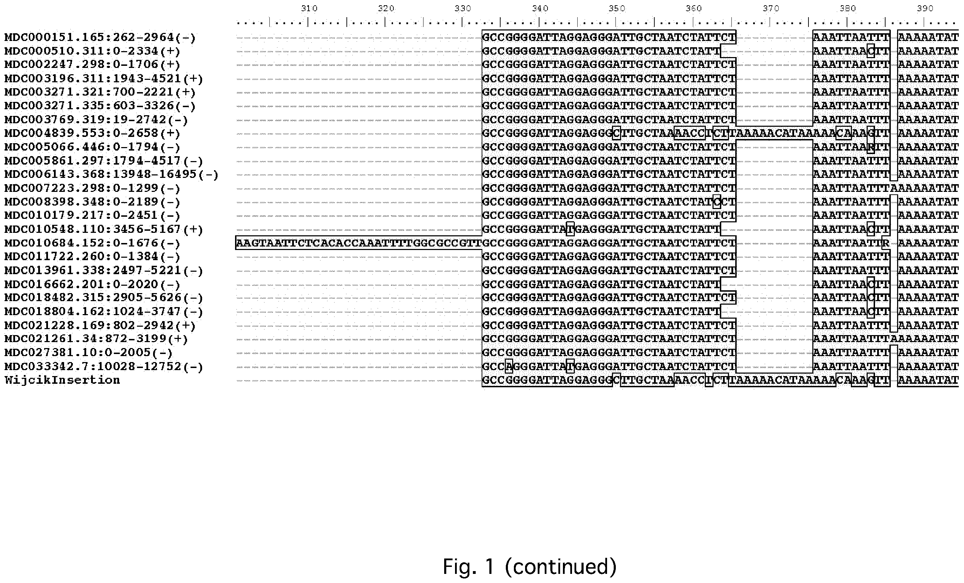

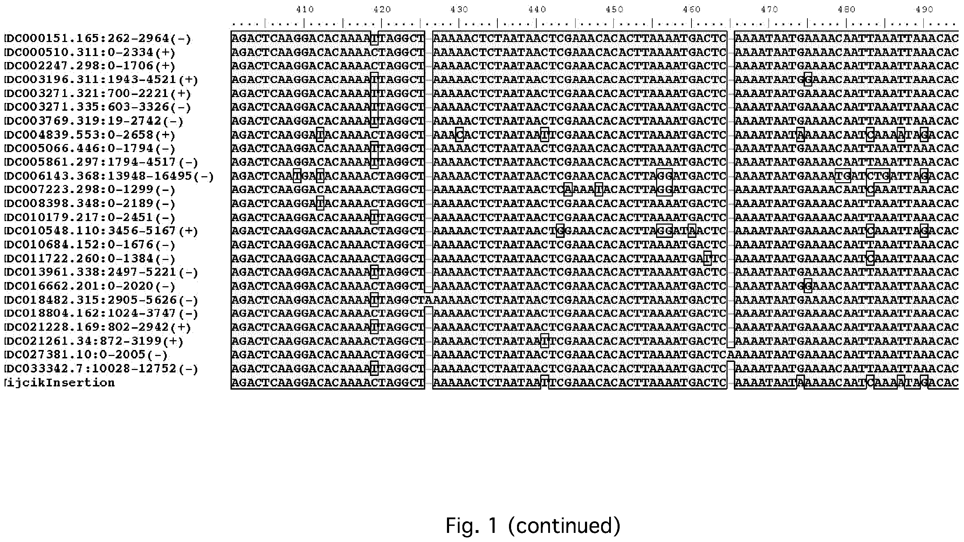

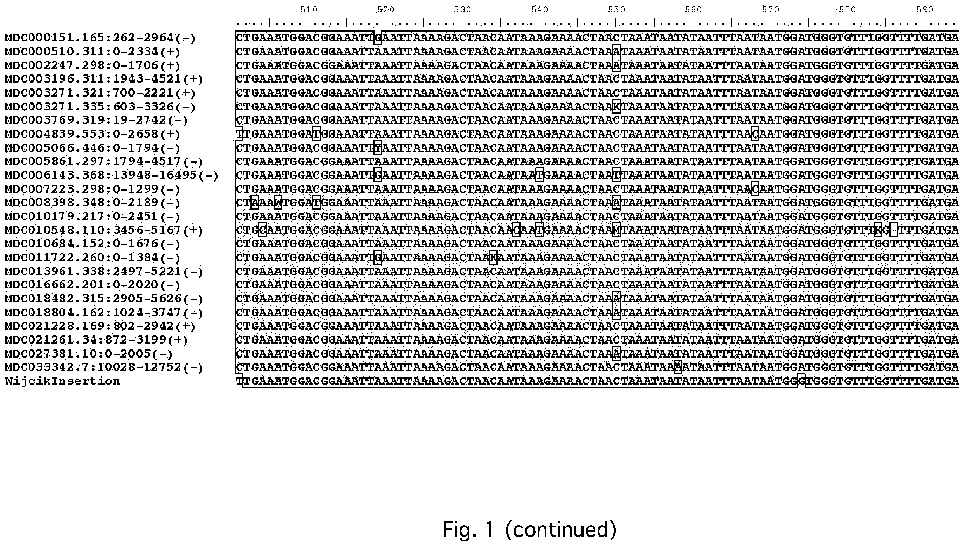

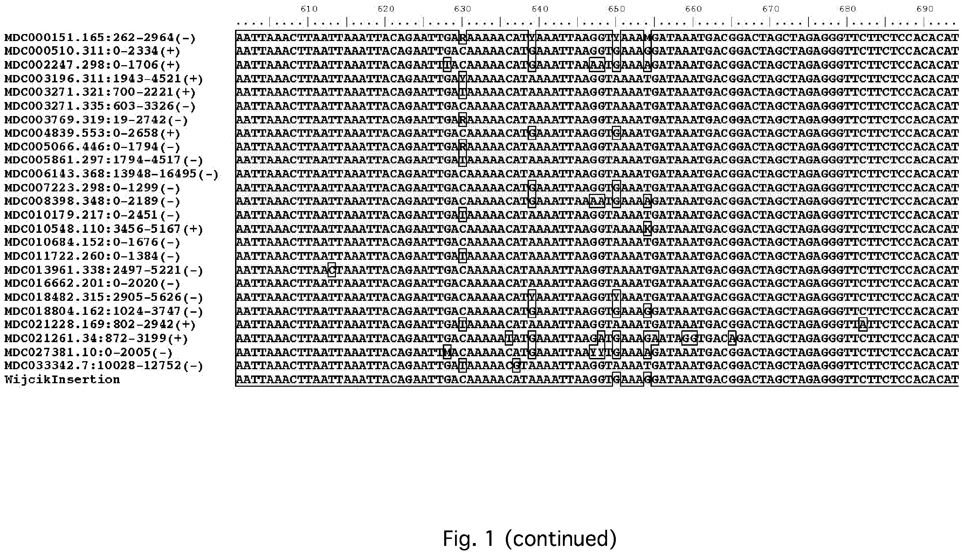

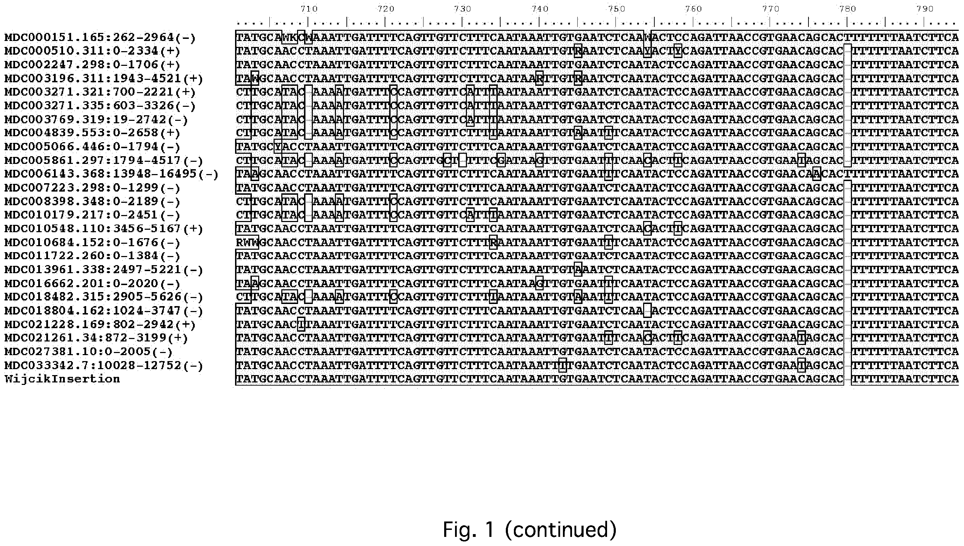

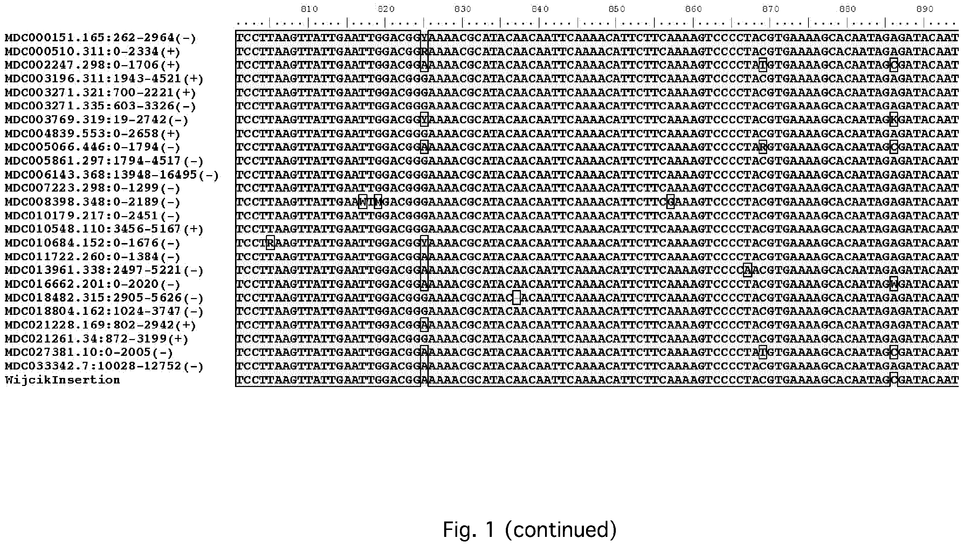

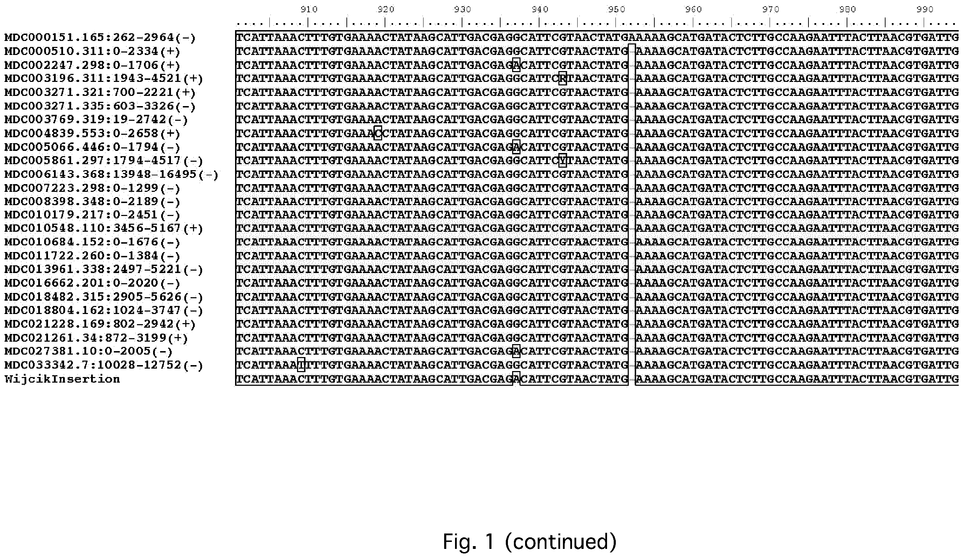

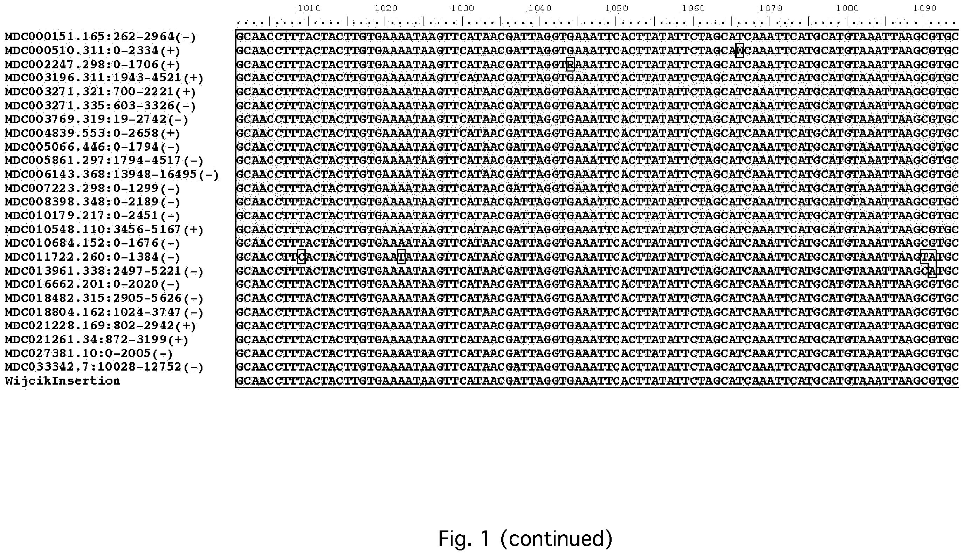

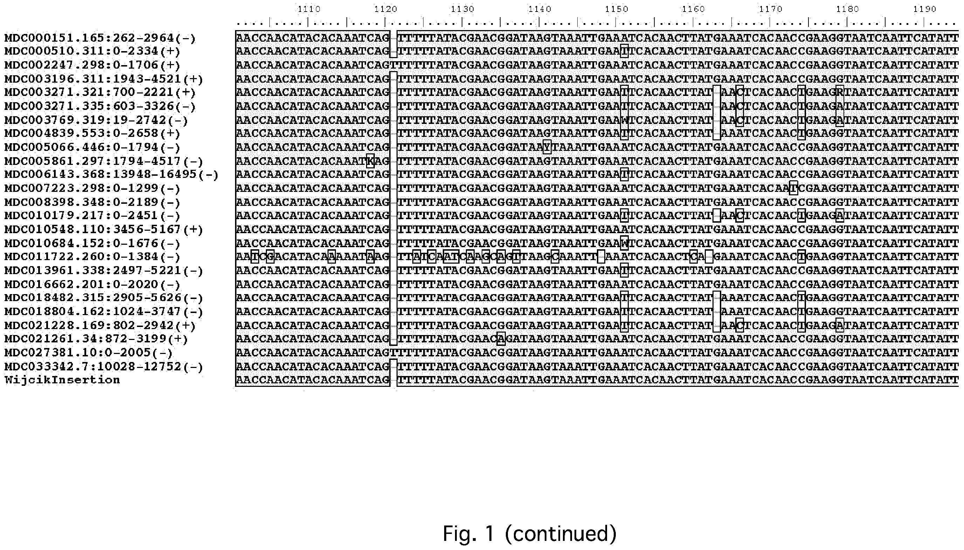

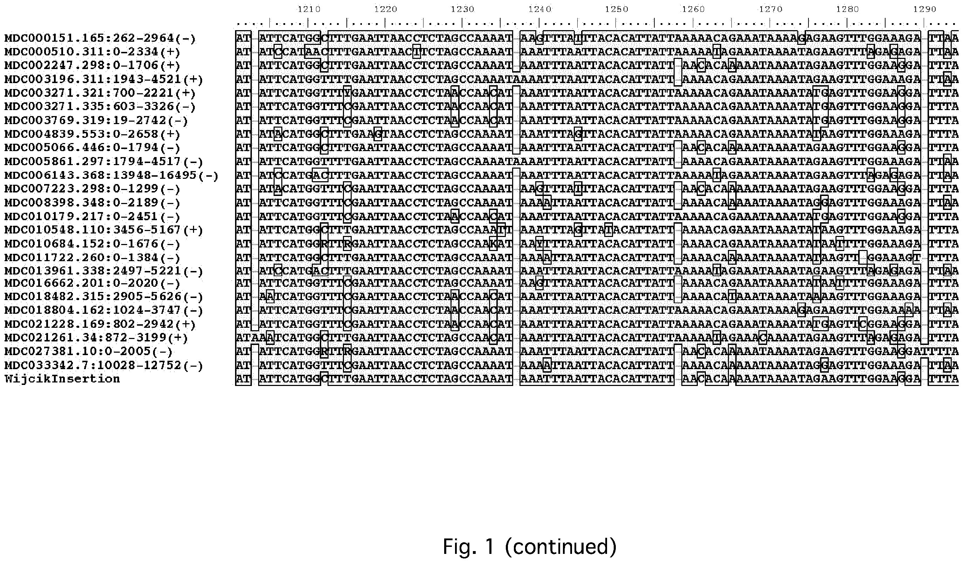

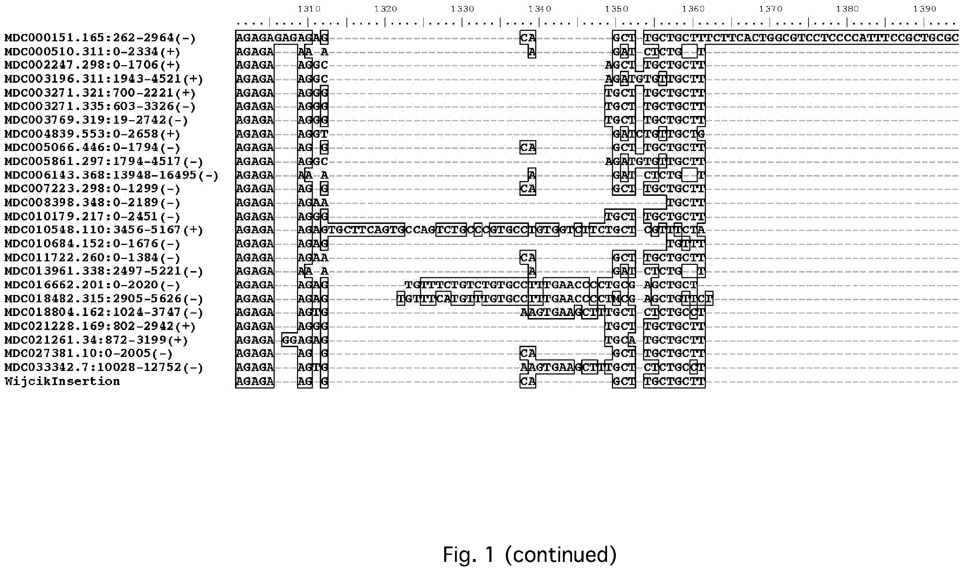

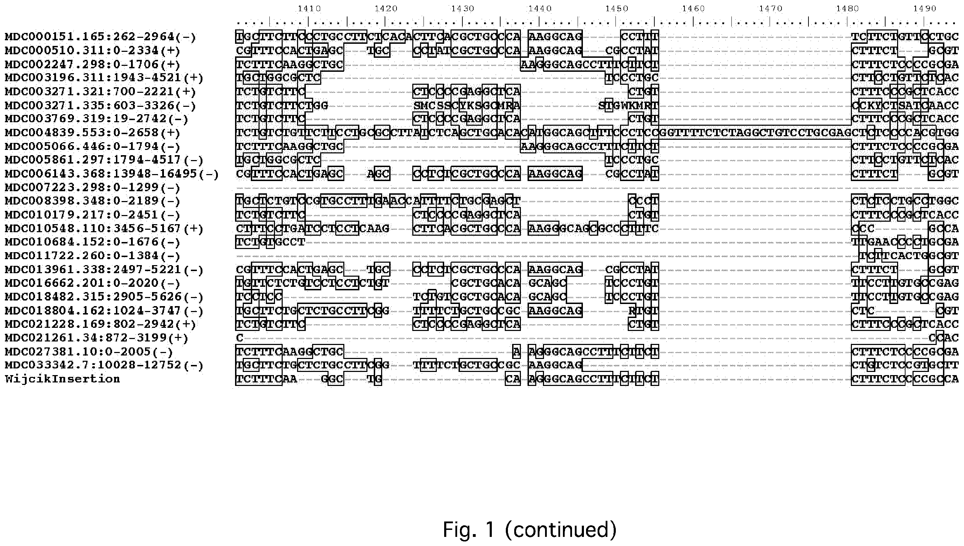

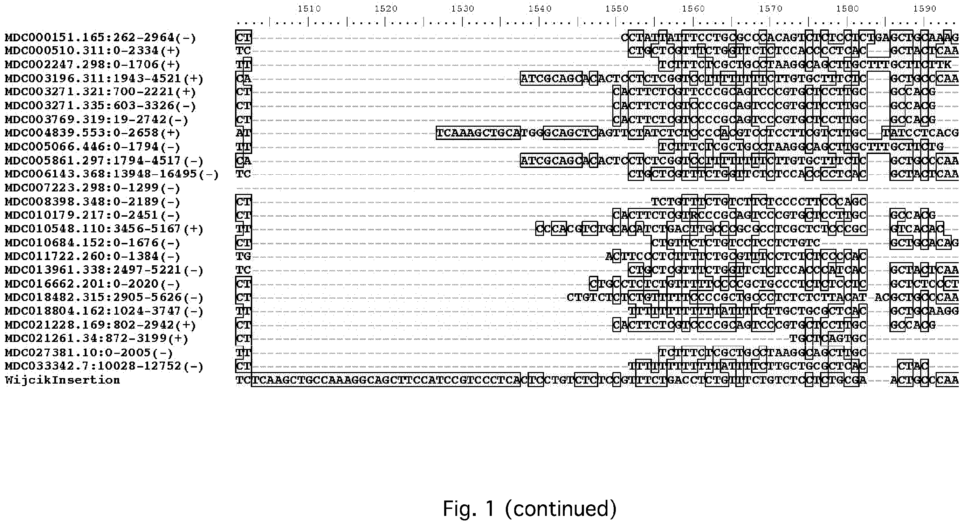

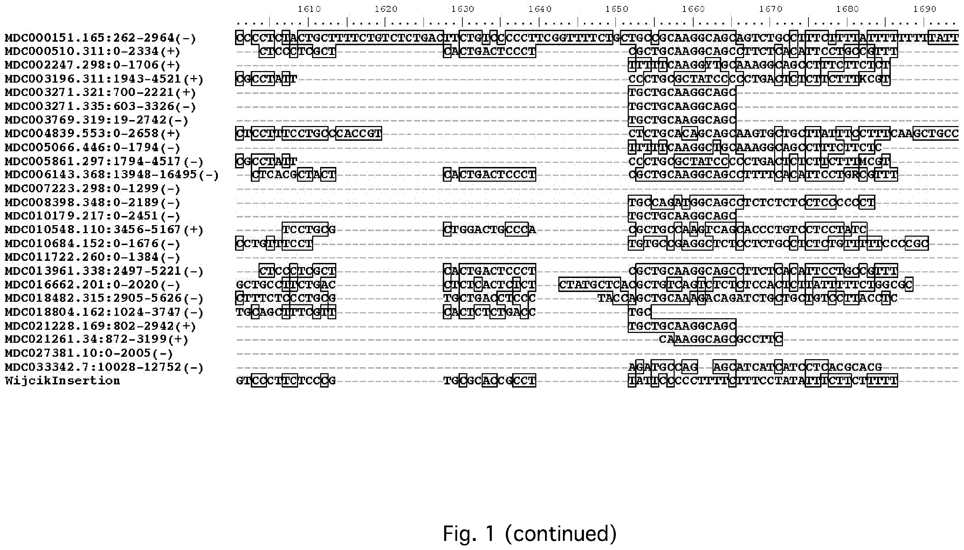

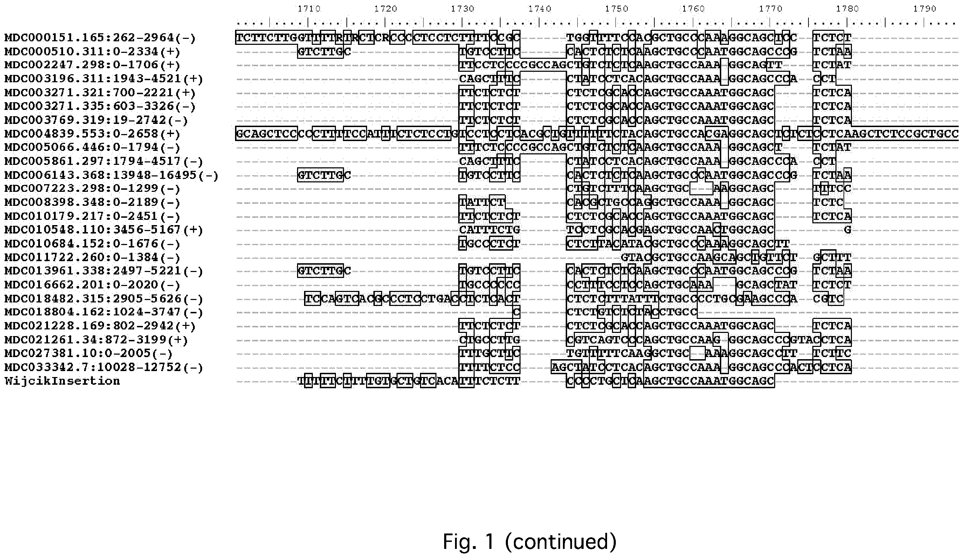

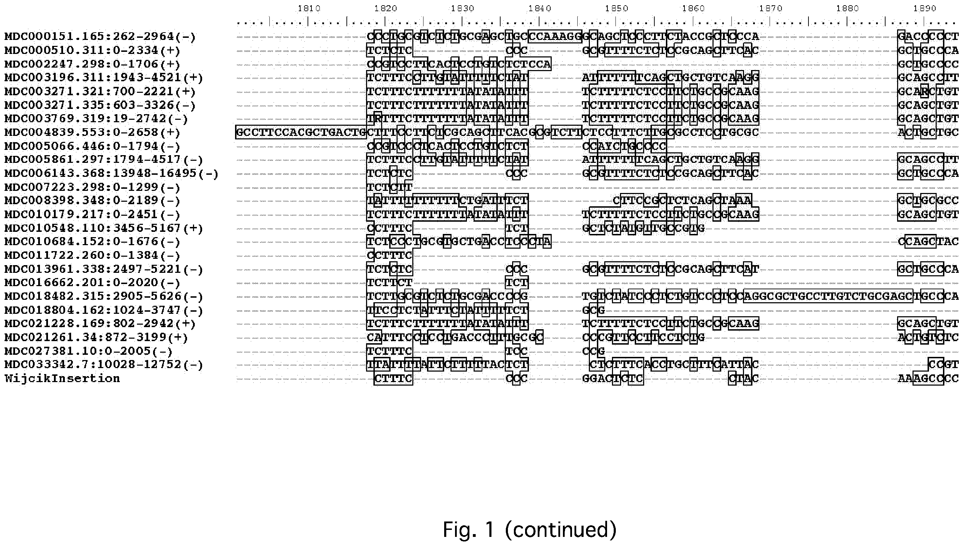

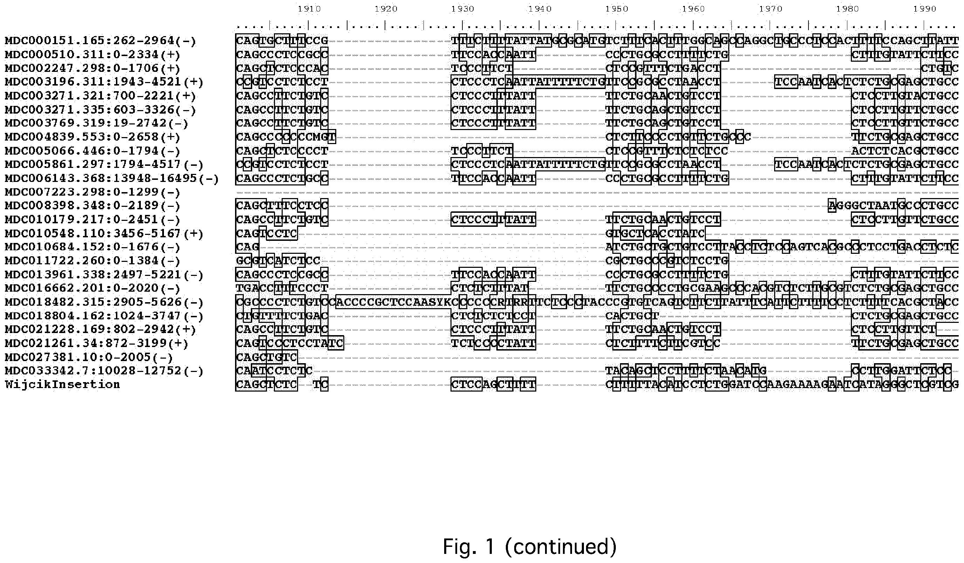

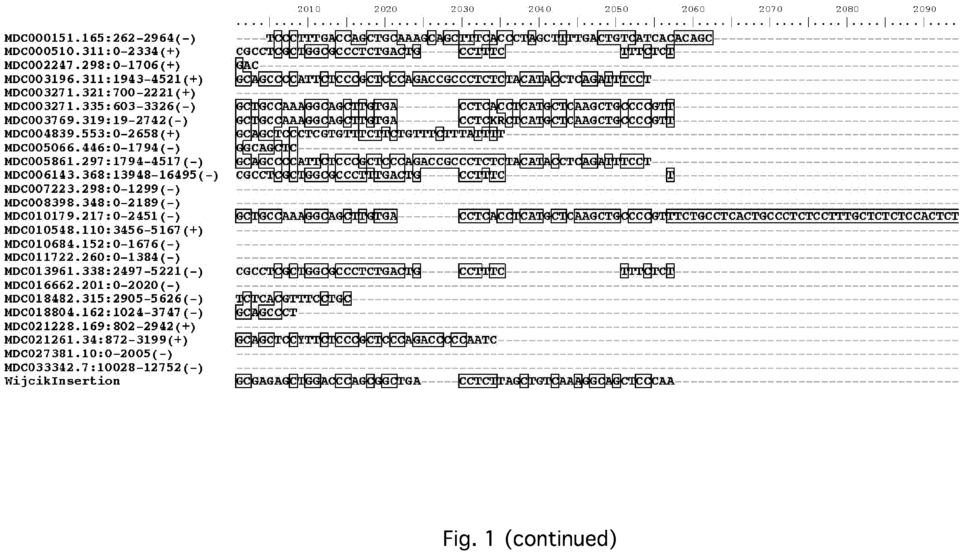

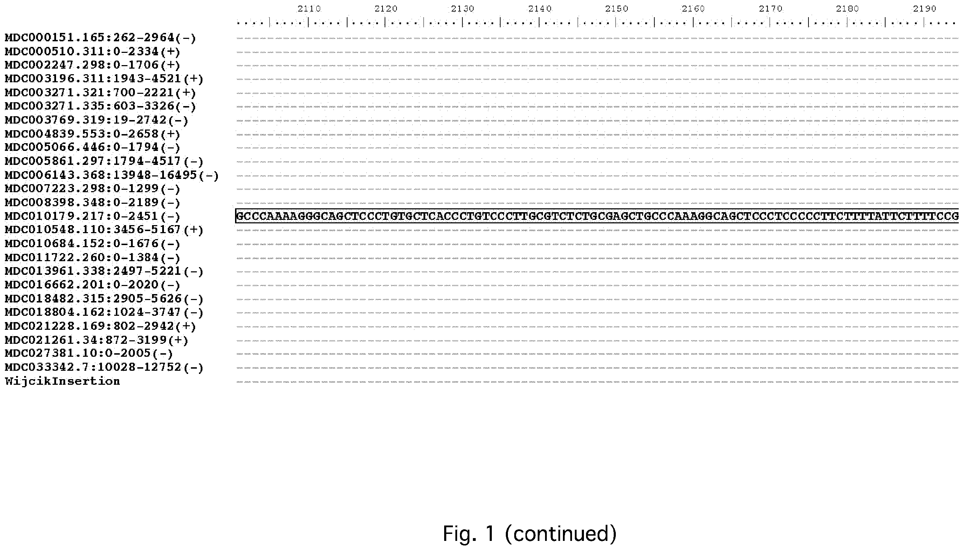

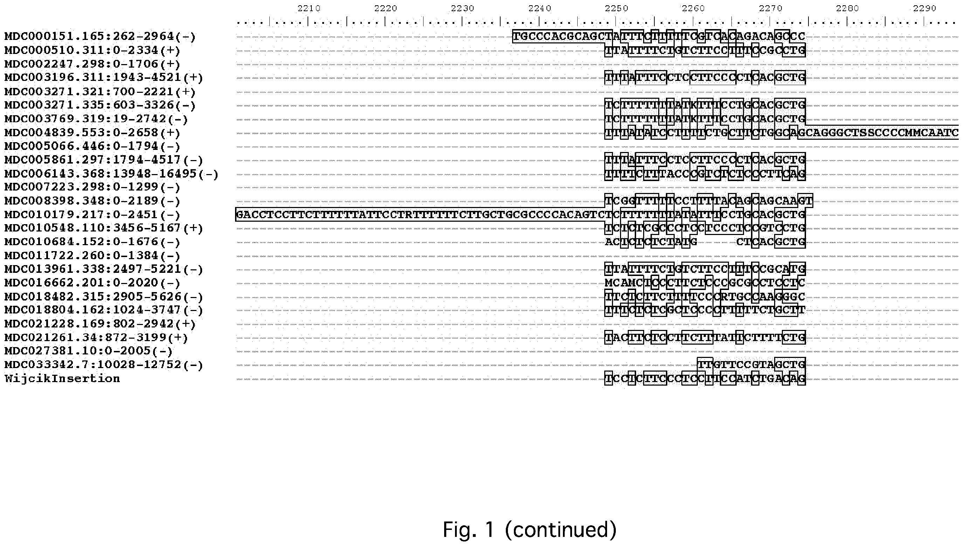

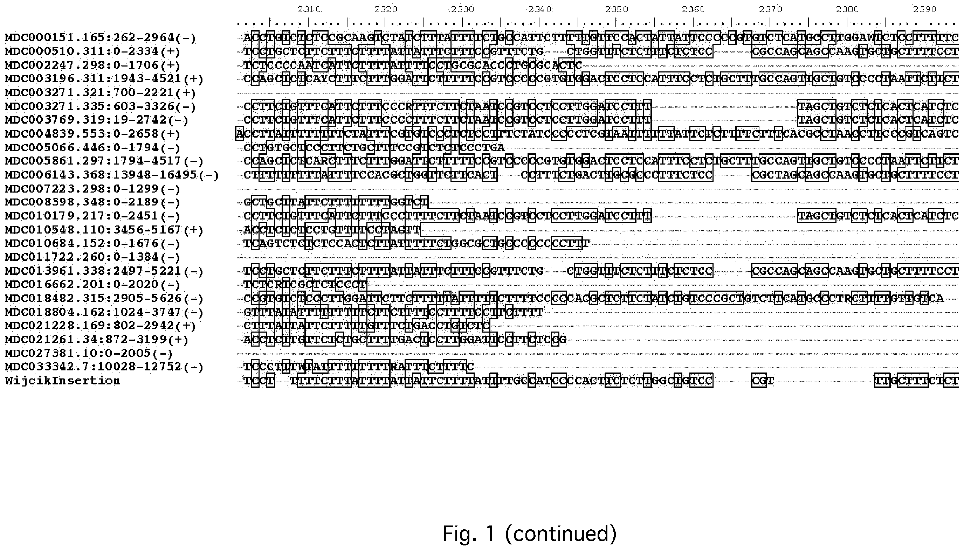

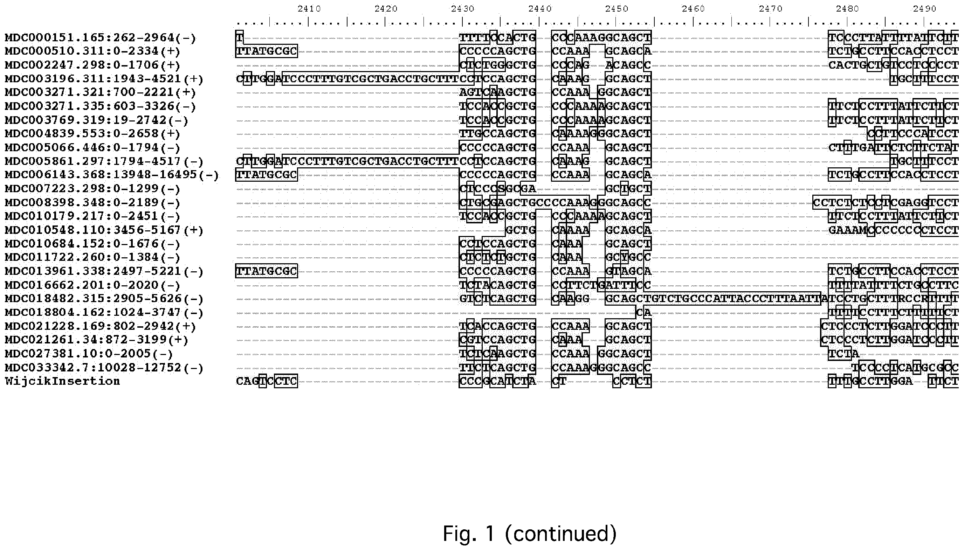

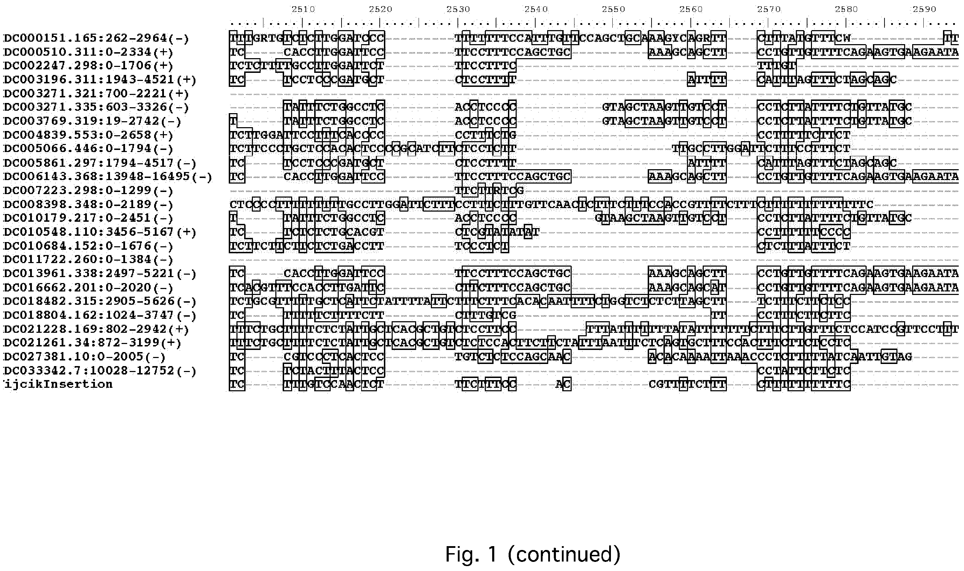

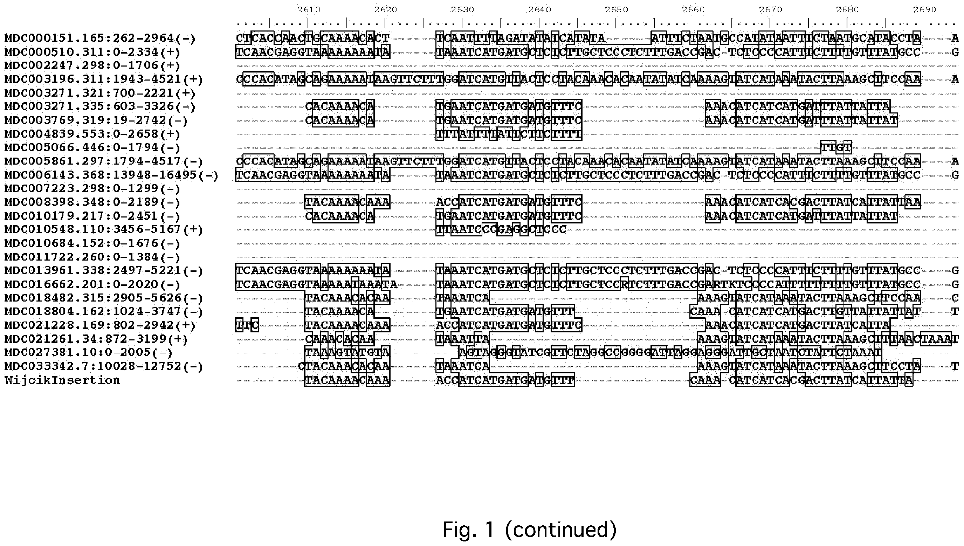

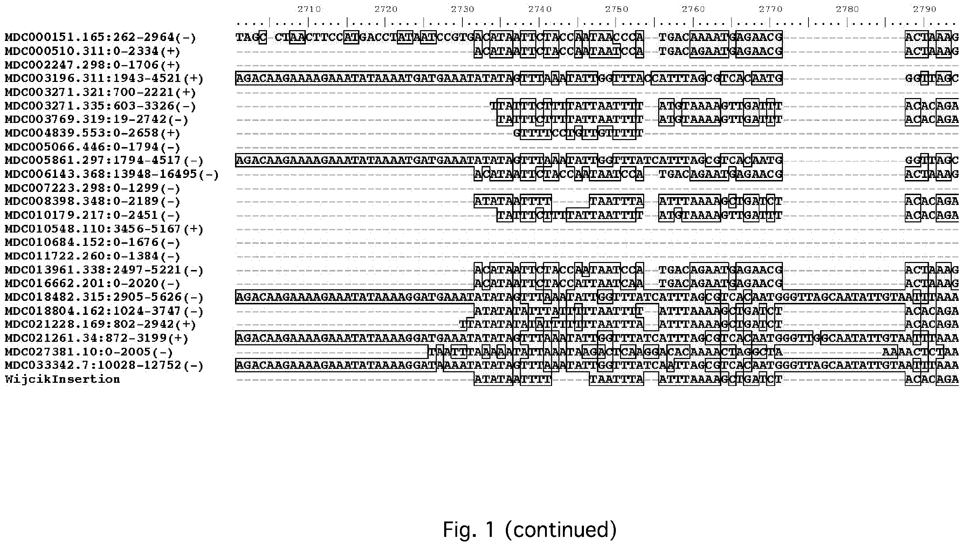

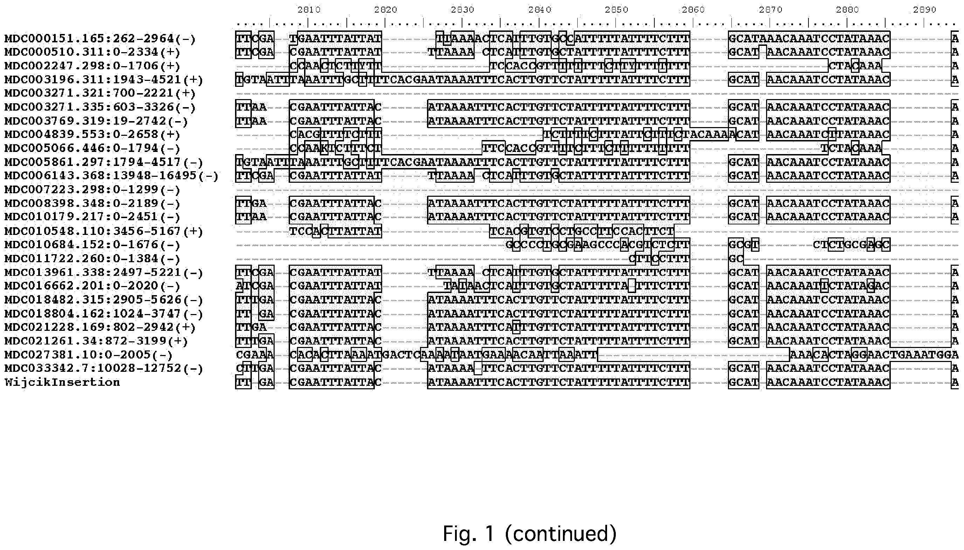

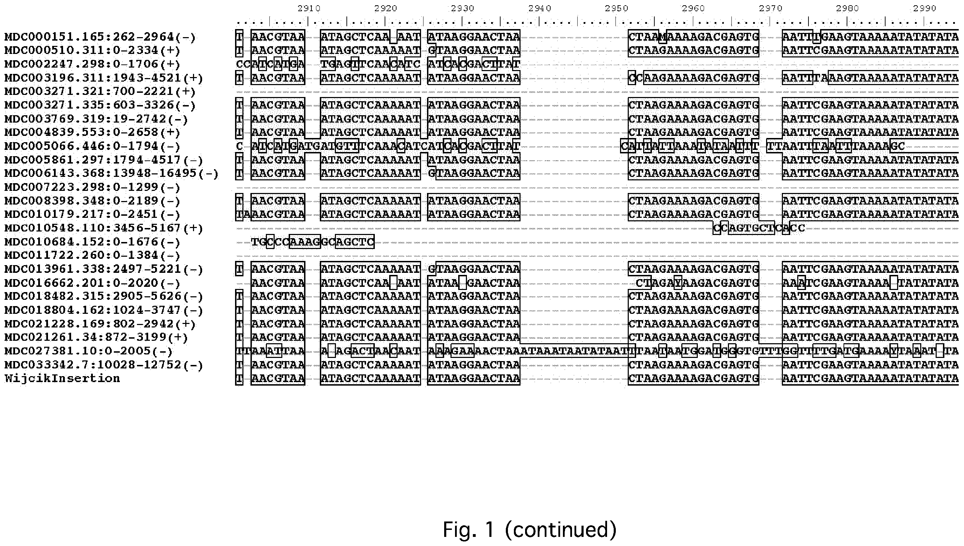

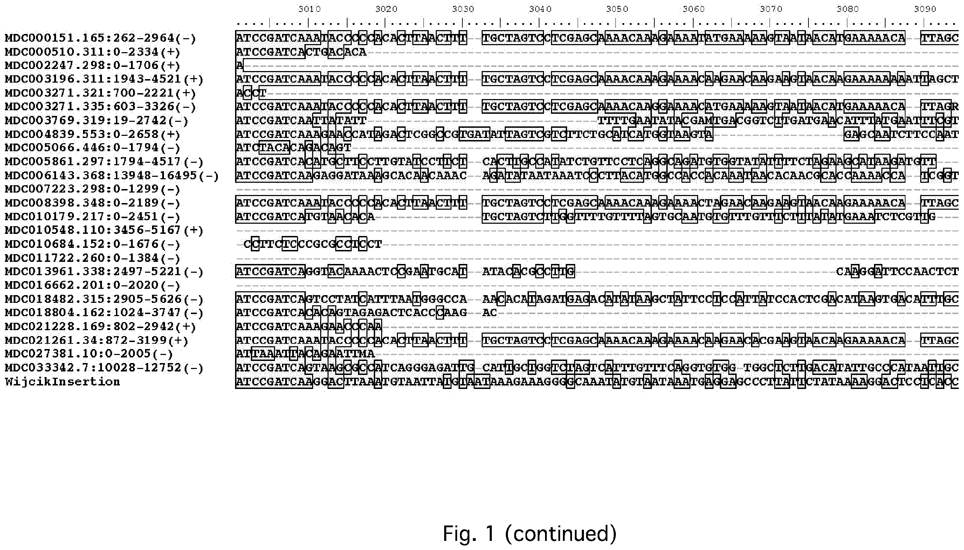

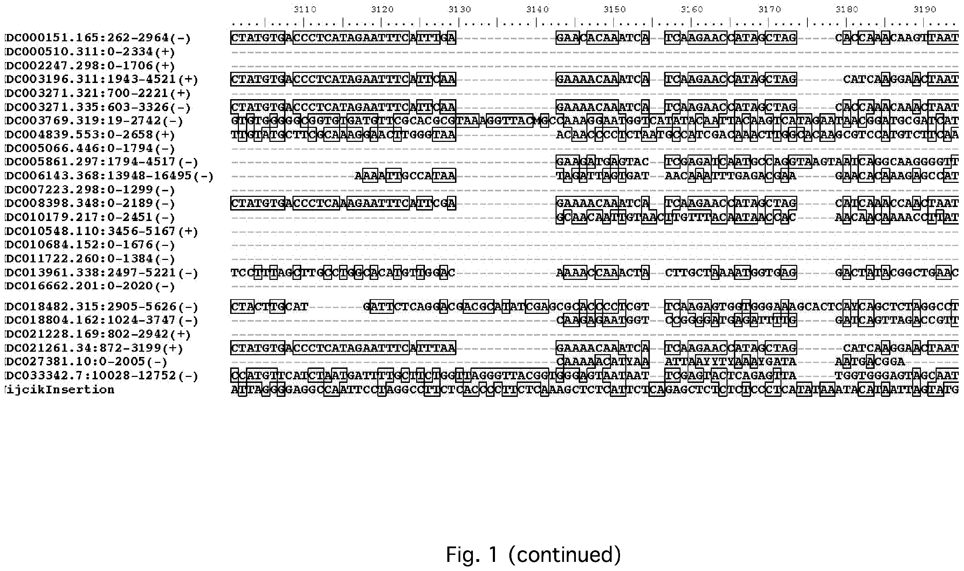

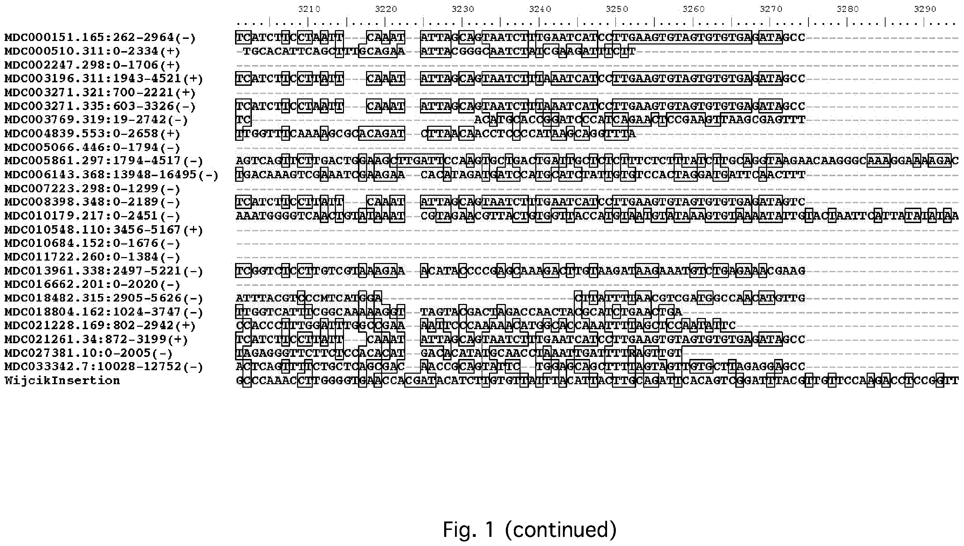

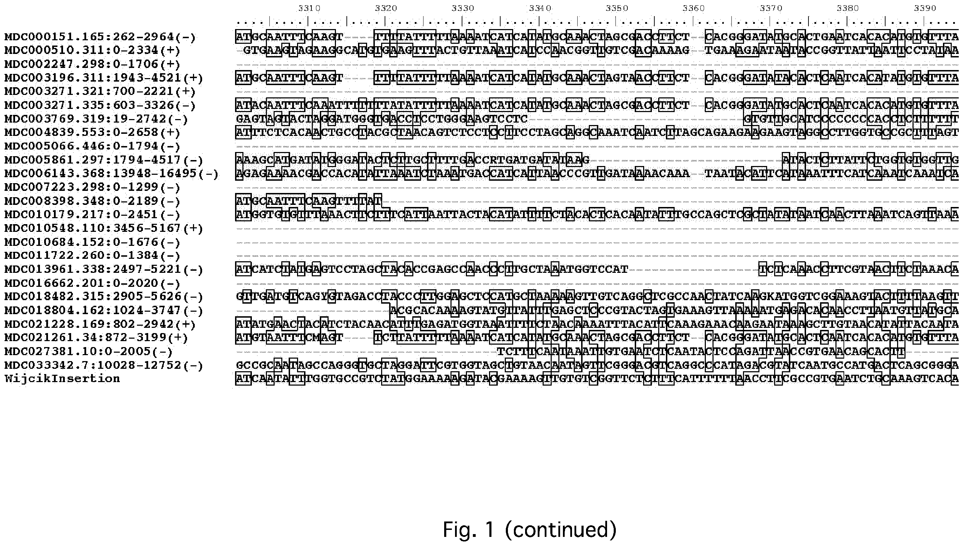

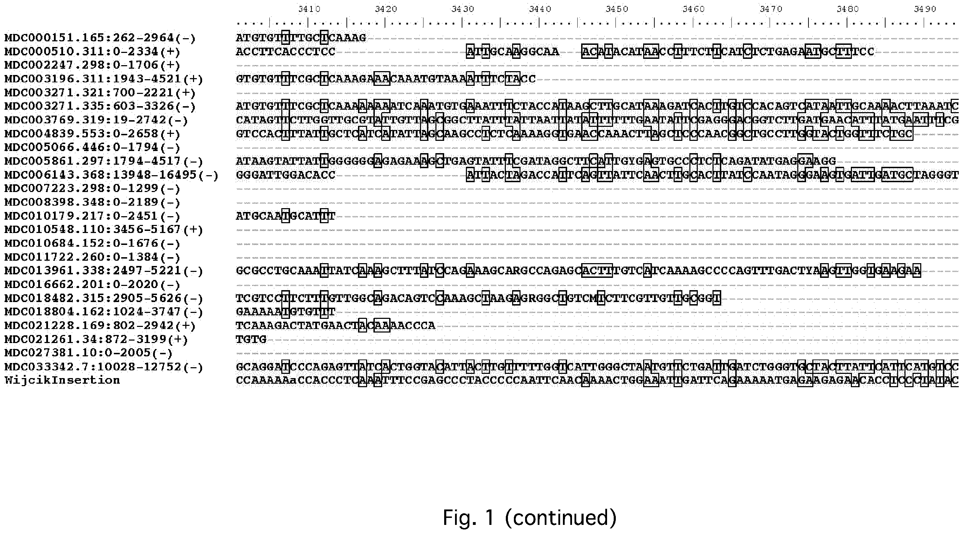

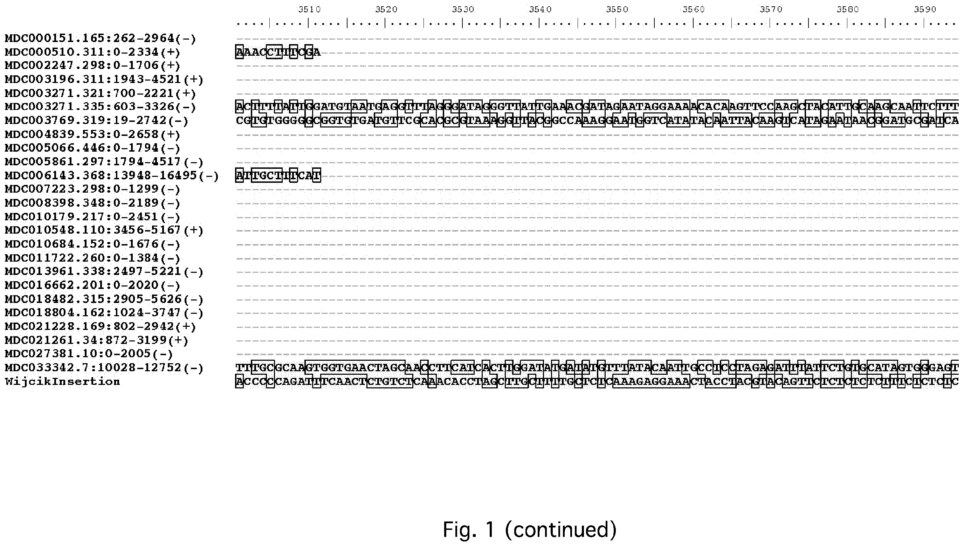

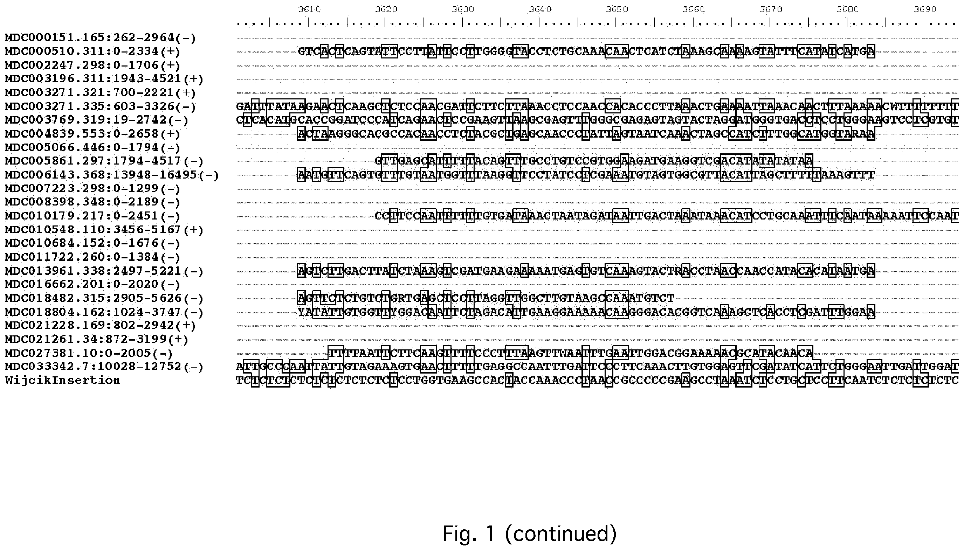

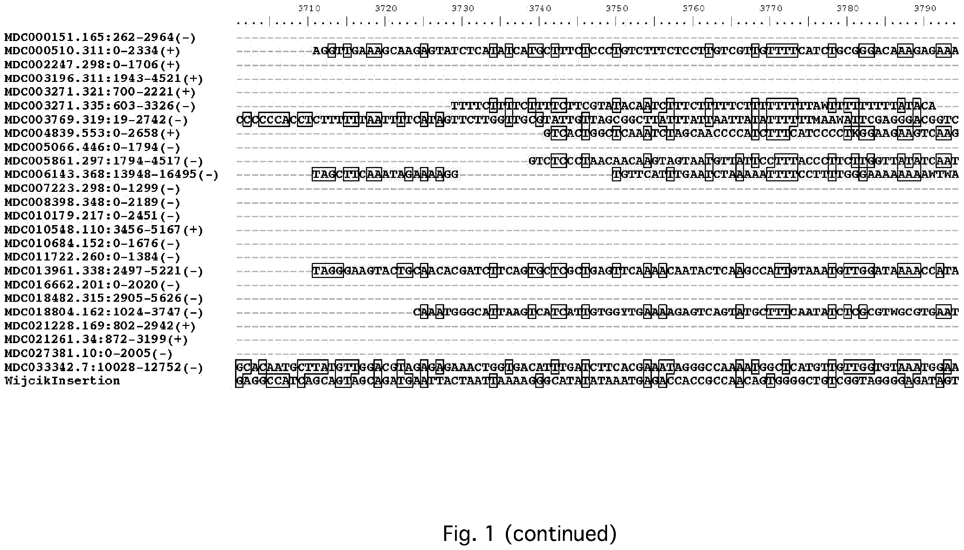

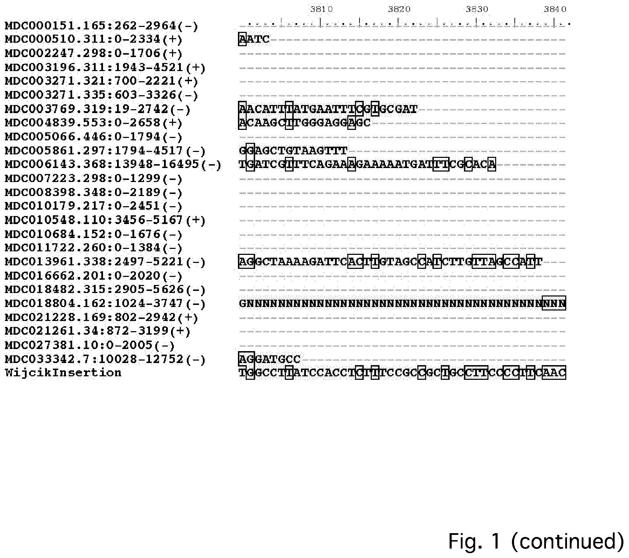

The final assembled sequences of 'McIntosh' and 'Wijcik' were aligned and visualized using SSAHA2 (Ning, 2001) and the Integrated Genomics Viewer (IGV) (Robinson, 2011). Figure 1 shows the genomic sequences of about 2 kb relating to the insertion region. The start of the insertion is visible at location 167 in the alignment, as the sequences are very similar from this point. The first conserved part of the insertion is around 1Kb, after which a more variable TC-rich region follows (starting from location 1310 in the alignment). The last approximately 160 bases of the insertion are more conserved again. The end of the 'Wijcik' insertion is at location 3012 of the alignment.

GlimmerHMM (Majoros, 2004) was used to identify ORFs in the 'Wijcik' sequence. The gene set from the homologous Co region of *Golden Delicious* was used as a reference (using BLAT; Kent, 2002). The gene predictions were annotated by performing a tBLASTx against the *Arabidopsis* protein database (Rhee, 2003). A conserved protein domain search was carried out using the NCBI's Conserved Domain Database (Marchler-Bauer, 2011).

For gene expression analysis, cDNA was synthesized from 1µg of RNA, using the VILO cDNA synthesis kit (Invitrogen). Specific primers were designed for each gene (see table 2).

**Table 2. Primers used for qPCR analysis**

| **Gene** | **forward primer (5'-3')** | **reverse primer (5'-3')** |
|---|---|---|
| MdCo27 | CTCCTAGAGGCCCGAGATG SEQ ID NO: 60 | TGCCGTGAGTTTCCTATTCC SEQ ID NO: 61 |
| MdCo28 | ACTGTGGAGGTCGGAAACC SEQ ID NO: 62 | AAGGCTCGTAAATGCCACAG SEQ ID NO: 63 |
| MdCo29 | GGATGGGGAAGGGATTAGAG SEQ ID NO: 64 | GGAGACTGAGGAAGGCACAT SEQ ID NO: 65 |
| MdCo30 | CCGTCTGTGGAACGACACTT SEQ ID NO: 66 | ATGCGTGTCATTTTGTGTGC SEQ ID NO: 67 |
| MdCo31 | CATAAAATGCCCCGAAAAGA SEQ ID NO: 68 | CAGAAGAATGAGCAGGGTGAG SEQ ID NO: 69 |
| MdCo32 | CCCCGAAGAGACAAGGAAAC SEQ ID NO: 70 | CCAGAATGACAGACCCCAAT SEQ ID NO: 71 |
| MdActin^{a} | TGACCGAATGAGCAAGGAAATTACT SEQ ID NO: 72 | TACTCAGCTTTGGCAATCCACATC SEQ ID NO: 73 |
| Md_4592:1:a^{b} | GTCGAAATGGTCAGCGGTAG SEQ ID NO: 74 | GCAATGGCAAACTCCACCTT SEQ ID NO: 75 |

| | | |
|---|---|---|
| a) MdActin is a housekeeping gene described by Li et al. (2008). b) Md_4592:1:a is a housekeeping gene described by Botton et al. (2011). | | |

The real-time PCR reaction conditions include incubation for 2 min at 50°C, followed by 2 min at 95°C and 40 cycles at 95°C for 15 s and 30 s at 60°C. All samples were loaded in triplicate. The results were analyzed using the method described by Pfaffl (Pfaffl, 20013). Actin (Li, 2008) and Md_4592:1:a (Botton, 2011) were found to be the most stably expressed house-keeping genes in the tissues we used for the expression analysis, therefore they were used as reference genes.

MdCo31 was amplified from RNA of 'Wijcik' using the SuperScript One-Step RT-PCR System (Invitrogen), cloned in the pENTR/D-TOPO vector using the TOPO kit (Invitrogen) and sequenced using M13 forward and M13 reverse primers.

### Identification of the Co gene

The inventors identified BAC clones covering the genomic Co region of 'Wijcik' and 'McIntosh' by screening the respective BAC libraries with specific markers (see table 3).

**Table 3. SSR markers used for screening the BAC libraries**

| **SSR name** | **Primer sequence** forward/reverse | | **SSR size (bp.) columnar**/ standard |
|---|---|---|---|
| Co04R09^{a} | f: 5'-TAGTGACATATACATGGTGCG-3' | SEQ ID NO: 76 | **156**/189 |
| | r: 5'-GTTGGAGAATGAGTGACGGC-3' | SEQ ID NO: 77 | |
| Co04R10^{a} | f: 5'-ACCTGGTTCCGGTACATAGC-3' | SEQ ID NO: 78 | **185**/203 |
| | r: 5'-AACCTTCCATGGCAGCAATC-3' | SEQ ID NO: 79 | |
| Co04R11^{a} | f: 5'-ACATCATGGTATGACAGAGGTG-3' | SEQ ID NO: 80 | **184**/172 |
| | r: 5'-TCTAAGCCTGTCAAGATGGC-3' | SEQ ID NO: 81 | |
| J8Co | f: 5'-TCGTTGCTCAATTACTGGCTA-3' | SEQ ID NO: 82 | **245**/232 |
| | r: 5'-AGGAATGCACAAATCCTCCA-3' | SEQ ID NO: 83 | |
| J9Co^{b} | f: 5'-TGTTGAGGGATTTTTCAGACG-3' | SEQ ID NO: 84 | **203**/201 |
| | r: 5'-GCACTCCCACCCCACTACTA-3' | SEQ ID NO: 85 | |
| J11Co^{b} | f: 5'-ACCCGAGACCTACCGCTTAT-3' | SEQ ID NO: 86 | **151**/156 |
| | r: 5'-TTTGAATGTGGTCCCTAGCC-3' | SEQ ID NO: 87 | |
| Co04R12^{a} | f: 5'-TTTATCTGACTAAGGGGAAGG-3' | SEQ ID NO: 88 | **195**/223 |
| | r: 5'-ATGGACTTGTATTCCTTAGGG-3' | SEQ ID NO: 89 | |
| J12Co^{b} | f: 5'-GCACCCCCAGATTTCAACT-3' | SEQ ID NO: 90 | **218**/216 |
| | r: 5'-GCTACTGCTGATGGCCTCTC-3' | SEQ ID NO: 91 | |
| J14Co^{b} | f: 5'-CTGAACATAACACCCTATACTTTGAA-3' | SEQ ID NO: 92 | **192**/139 |
| | r: 5'-GACTTCAAACCTGAAGGGATAAAA-3' | SEQ ID NO: 93 | |
| Co04R13^{ac} | f: 5'-ATTTTCCCTCTCTTCTGTTGC-3' | SEQ ID NO: 94 | **237**/235 |
| | r: 5'-TCTTGGAAAGACGTGGCACG-3' | SEQ ID NO: 95 | |

| | | | |
|---|---|---|---|
| a) SSR marker was used in Baldi et *al.* (Baldi, 2012). b) Homologue of SSR marker was described by Moriya et *al.* (Moriya, 2012). c) Homologue of SSR marker was described by Bai et *al.* (Bai, 2012). | | | |

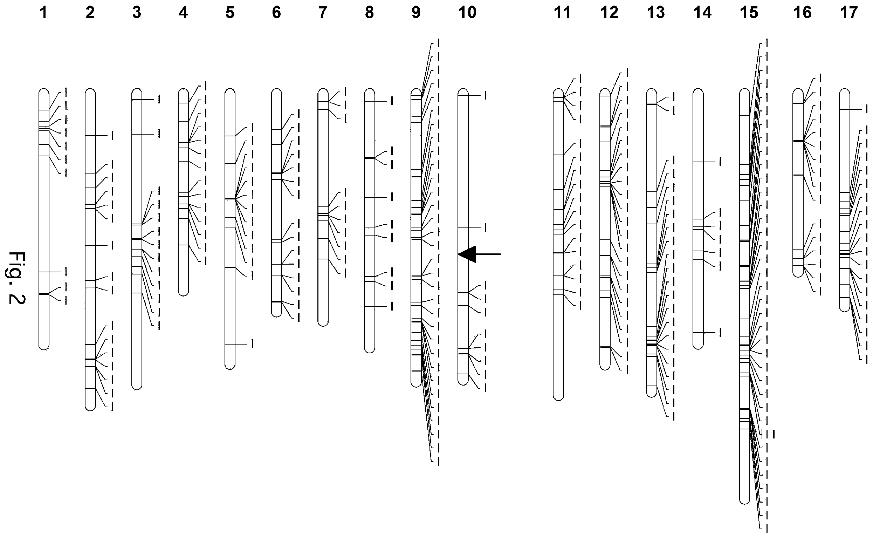

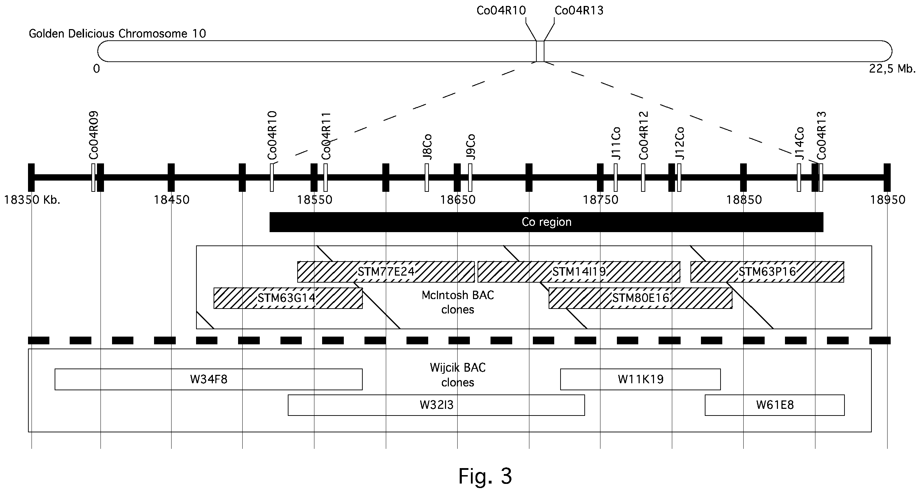

Only BAC clones in coupling phase with Co were selected during BAC screening. The BAC ends of all positive clones were sequenced and the resulting sequences were used to anchor the clones on the *Golden Delicious* genome sequence, creating a minimum tiling path of BAC clones spanning the complete Co region. A small region of 13 kb was not covered by the 'McIntosh' BAC library (fig. 3). Fig. 2 shows an overview of the positions of sequences homologous to the 'Wijcik' insertion in the *Golden Delicious* genome. The first conserved part of the 'Wijcik' insertion was blasted on the *Golden Delicious* genome. More than 250 highly similar sequences were found. The locations of these sequences on the 17 chromosomes of *Golden Delicious* are indicated ('l'). The deduced position of the 'Wijcik' insertion (not present in *Golden Delicious*) is indicated with an arrow on chromosome 10.

The selected BAC clones were sequenced using 454 sequencing with an average coverage of 100x. After cleaning and trimming of the reads, they were assembled, resulting in 25 contigs for the complete Co region of 'Wijcik' and only 6 contigs for the 'McIntosh' BAC clones. In order to join the contigs, primers were designed on the termini and used for Sanger sequencing (see table 1). This way, the number of contigs for the 'Wijcik' Co region could be reduced to WijcikContig1 (WiC1) and WijcikContig2 (WiC2) with a length of 354 and 34 kb respectively. The two contigs could not be joined as they were found to be separated by a long sequence of repetitive DNA. Fig. 3 depicts the BAC contig construction. Chromosome 10 from *Golden Delicious* is shown at the top of the figure. SSR markers delimiting the Co region (Co04R10 and Co04R13) are indicated on the chromosome. The Co homologous region from *Golden Delicious* is shown below, together with all the SSR markers used for BAC screening. The BAC clones used for construction of the consensus sequence covering the Co region in 'McIntosh' and 'Wijcik' and their locations on the homologous region from *Golden Delicious* are displayed at the bottom of the figure.

The same strategy was used to sequence the *'McIntosh'* region. Additionally, in order to sequence the 13 kb segment that was not covered by the 'McIntosh' BAC clones, primers were designed based on the homologous 'Wijcik' region and used to amplify overlapping fragments from 'McIntosh' genomic DNA (see table 4).

**Table 4. Primers used to sequence the part of the 'McIntosh' Co region not covered by the BAC library**

| **Primer name** | **Sequence (5'-3')** | |
|---|---|---|
| JseqSTMG1f | AACTGTCAGGAATCGGAATAGG | SEQ ID NO: 96 |
| JseqSTMG1r | TCAACATCATTCTTGCCATCTT | SEQ ID NO: 97 |
| JASSTMG2f | TGTAAACGTGCTTTCAGCAAA | SEQ ID NO: 98 |
| JASSTMg1r | AGAAGAGTTCCGGTGAGCAA | SEQ ID NO: 99 |
| JseqSTMG2f | CACGTGTCAATGTCAGAATGAA | SEQ ID NO: 100 |
| JseqSTMG2r | CGTCCACAGAGTAGAGTTCTCA | SEQ ID NO: 101 |
| JASSTMg3f | TCAGGAAACAATGGGGTTCT | SEQ ID NO: 102 |
| JASSTMg2r | TGGTGAAAATGATGGGGTTT | SEQ ID NO: 103 |
| JseqSTMg3f | TTGGCCCAAAATCCAAATAA | SEQ ID NO: 104 |
| JseqSTMg3r | CTTCAAGTTTTCATGGGTGATG | SEQ ID NO: 105 |
| JASSTMg4f | TCCATTGGAGGAAACAAAGC | SEQ ID NO: 106 |
| JASSTMg3r | TTGTGGTCTCCAGTTCAACG | SEQ ID NO: 107 |
| JseqSTMg4f | TGCATCGAATCCTCAAATCA | SEQ ID NO: 108 |
| JseqSTMg4r | ATGGTCCGTATGGAAGGTGA | SEQ ID NO: 109 |
| JASSTMg5f | GGCGTCAATATTGGTGATCC | SEQ ID NO: 110 |
| JASSTMg4r | AAACTAGCATAATAGATCGAAATCTCA | SEQ ID NO: 111 |
| JseqSTMg5f | TGCTCTCAATTCGACTGTGG | SEQ ID NO: 112 |
| JseqSTMg5r | GTTCTCGCGATCTCTTCAGG | SEQ ID NO: 113 |
| JASSTMg6f | ACGCATCATGCATCAAACAG | SEQ ID NO: 114 |
| JASSTMg5r | CCAATATGGGTGCCTCGTTA | SEQ ID NO: 115 |
| JseqSTMg6f | GACTTGCTGCTTCGTGCATA | SEQ ID NO: 116 |
| JseqSTMg6f2 | ACGTCCACGGAATAGAGGAG | SEQ ID NO: 117 |
| JseqSTMg6r | TTTTAAACCCAAGACCTCCTCA | SEQ ID NO: 118 |
| JASSTMg7f | GACTCAACAACAACAACAACAACA | SEQ ID NO: 119 |
| JASSTMg6r | GGAAGTCAAGCCGATACCAC | SEQ ID NO: 120 |
| JseqSTMg7f | AGGCCGACTGAAGAATTTGA | SEQ ID NO: 121 |
| JseqSTMg7r | CTTACCCTGGCATTGCTCTC | SEQ ID NO: 122 |
| JASSTMg7r2 | TCTAACAAAGTTGAGACTACTTTTCCA | SEQ ID NO: 123 |

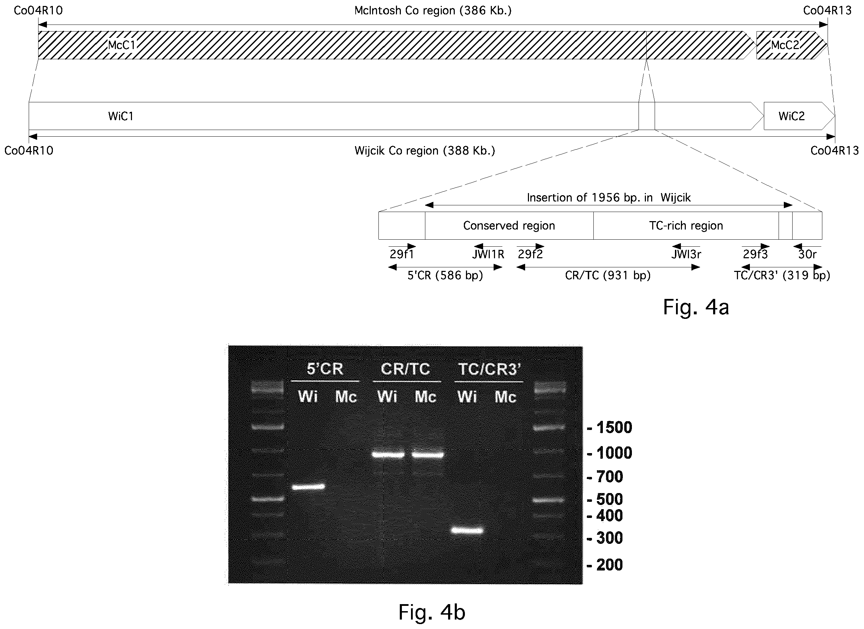

Similarly to 'Wijcik', this approach resulted in the assembly of two contigs, McIntoshContig1 (McC1) and McIntoshContig2 (McC2), with a length of 352 and 34 kb respectively, interrupted by a long sequence of repetitive DNA. Figures 4A and 4B show the BAC sequence assembly and identification of 'Wijcik' insertion. Fig. 4A illustrates that assembly of the BAC clones covering the Co region resulted in two contigs both in 'McIntosh' ('McC1' and 'McC2') and 'Wijcik' ('WiC1' and 'WiC2'). The location of the insertion in 'McIntosh' and 'Wijcik' is indicated on McC1 and WiC1. Primers used to detect the presence of the insertion in 'Wijcik' and 'McIntosh' and the names of the corresponding amplification products are indicated on the 'Wijcik' insertion. Figure 4B shows the specific amplification of the insert sequence from genomic DNA of 'Wijcik' ('Wi') and 'McIntosh' ('Mc'). The different amplification products are indicated above each lane. When one primer was designed outside and one inside the insert sequence, amplification products (fragments '5'CR' and 'TC/Cr3") were only detected in 'Wijcik'. When both primers were designed within the insert sequence, an amplification product (fragment 'CR/TC') was detected in 'Wijcik' as well as in 'McIntosh'.

The sequences obtained from the positive BAC clones enabled the inventors to directly compare the genomic Co region of 'McIntosh' and 'Wijcik'. As 'Wijcik' originated from a somatic mutation in 'McIntosh' (Fisher, 1969), the sequences were expected to be almost identical, apart from the mutation that lead to columnar growth.

Indeed, a very small number of differences were found between the Co region sequence of 'Wijcik' and 'McIntosh', most of which could be attributed to sequencing and assembly errors. The only difference found between the two sequences is an insertion of 1956 bp in 'Wijcik' (Fig. 4A). Interestingly, this insertion also falls within the putative Co region described by Moriya et al. (2012) using a different cross population. This strongly suggests that the 1956 bp insertion found in 'Wijcik' is responsible for the columnar phenotype.

In order to confirm the presence of the insertion in the 'Wijcik' genome and to exclude possible artifacts occurred during BAC library construction or assembly of the 454 reads, selective amplifications were performed on genomic DNA of 'McIntosh' and 'Wijcik' using specific primers (see table 5).

**Table 5. Primers used for 'Wijcik' insert amplification**

| **Primer name** | **Primer sequence (5'-3')** | |
|---|---|---|
| 29f1 | CCTGGATCTAAAAGCCCCATA | SEQ ID NO: 124 |
| JWI1r | AACCAAACACCCACCCATTA | SEQ ID NO: 125 |
| 29f2 | GCTAGAGGGTTCTTCTCCACA | SEQ ID NO: 126 |
| JWI3r | GTAGGAGAGTCCGGGGAAAG | SEQ ID NO: 127 |
| 29f3 | TGATCTACACAGACAGTTTTGACG | SEQ ID NO: 128 |
| 30r | GCTTTGAGAAGGGGTGAGAA | SEQ ID NO: 129 |

When one primer was designed on the flanking region of the insert sequence and one inside the insert sequence itself, a PCR product was obtained only when 'Wijcik' genomic DNA was used as a template (Fig. 4B). Interestingly, when amplification was performed using primers that were both designed inside the insert sequence, a PCR product was obtained in 'McIntosh' as well (Fig. 4B), suggesting the presence of one or more copies of the insert sequence in the apple genome. By performing a BLAST search, over a hundred of sequences homologous to the 'Wijcik' insertion were found distributed on *Golden Delicious* reference genome. No description of a similar sequence could be found either in plant gene databases, or in genomes of closely related *Rosaceae* species, like strawberry and peach, indicating that the inserted DNA element in 'Wijcik' Co region is specific to apple and has evolved recently.

Furthermore, comparison of sequences by alignment showed that the sequence of the insertion in 'Wijcik' is only partially conserved, as in *Golden Delicious* a high similarity was found only in the first half of the sequence, followed by a less conserved TC-rich region and the last 160 bases again highly conserved (Fig. 1). When the first conserved part of the insert sequence was aligned against the *Golden Delicious* genome, a total of 257 highly similar sequences were found (Fig. 2).

Taken all together, our data show that the insertion in 'Wijcik' Co region evolved recently in apple genome and spread by an unknown mechanism, since such element does not belong to the group of DNA transposable elements, as it lacks the terminal inverted repeats (TIR) that are normally present in this class of molecules (Bennetzen, 2000). The observation that both ends of the insert sequence seem to be highly conserved among the different homologues found in *Golden Delicious* might indicate that these parts of the insertion are important for its transposition.

The insertion found in 'Wijcik' occurred in intergenic regions and the insert sequence itself does not contain any open reading frame, raising the question on how the insertion causes the columnar phenotype of 'Wijcik'. A possibility is that the insertion has an effect on the expression of neighboring genes. To test this hypothesis, the expression of all genes located within a region of 25 kb upstream and downstream of the insertion was compared between 'McIntosh' and 'Wijcik'. As columnar plants differ from wild type trees in branch development, both leaves and buds were used as starting material.

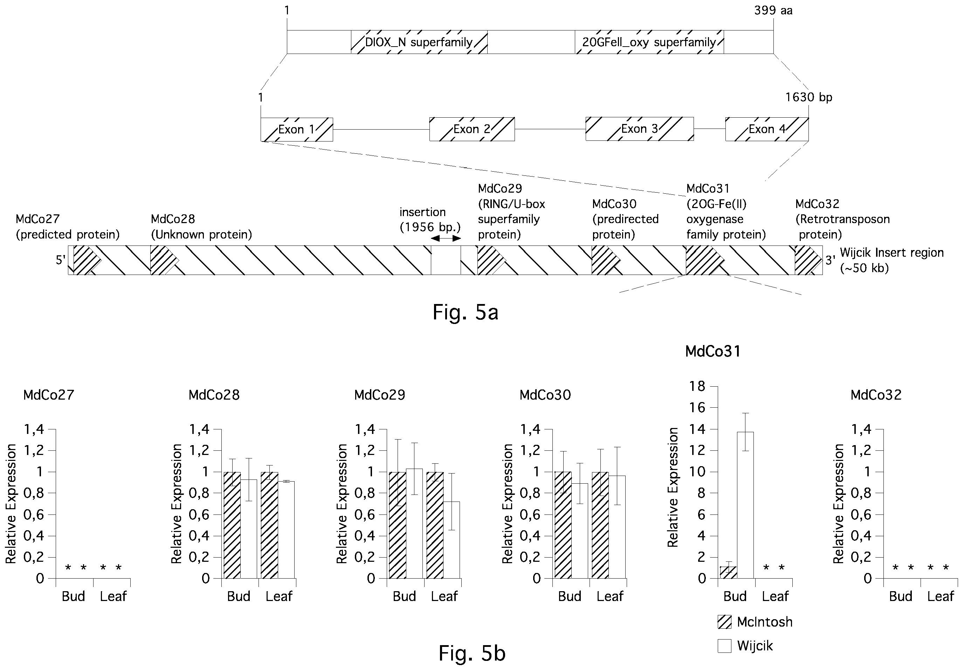

Figures 5A and 5B show the annotation of the insert region and qPCR results. Fig. 5A shows the 'Wijcik' 50 Kb region containing the insertion and structure of MdCo31. The conserved DIOX_N superfamily and 2OG-Fell_oxygenase domains together with the predicted exons are shown (grey boxes). The position of the predicted genes within the candidate region is indicated by arrows. Figure 5B depicts the qPCR analysis of the predicted genes within the candidate region in bud and leaf material from 'McIntosh' and 'Wijcik'. Expression levels are calculated based on three biological replicates and the standard deviations are indicated by error bars. Gene expression in 'Wijcik' (right bars) is normalized against the expression of the corresponding gene in 'McIntosh' (left bars). Asterisks indicate the samples for which the expression level is below the limit for reliable quantification.

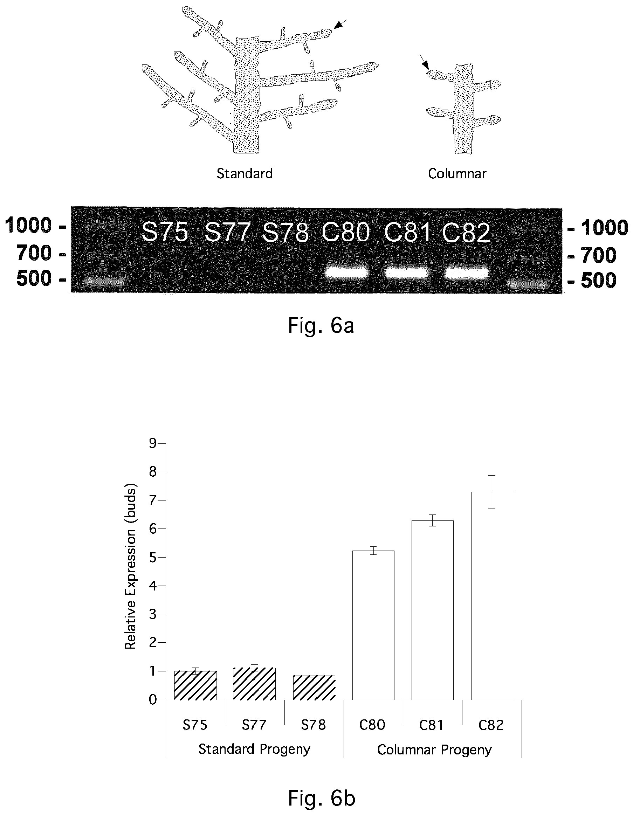

Six genes were predicted within the 50 kb region (Fig. 5A), of which only MdCo31 was found to be differentially expressed between 'Wijcik' and 'McIntosh' buds (Fig. 5B; supplementary data, SEQ ID NO: 1 and 2 for nucleotide and amino acid sequence). The expression of MdCo31 in axillary buds of 'Wijcik' was found to be ca.14-fold higher than in 'McIntosh' buds, strongly suggesting a role for this gene in controlling the columnar phenotype, since the increased expression of MdCo31 in 'Wijcik' is in accordance with the dominant character of the trait. Moreover, the overexpression of MdCo31 appeared to be tissue-specific, as no difference was detected in leaves (Fig 5B). This is well in accordance with the supposed function of controlling shoot development. If the presence of the 1956 bp insertion and the expression difference of MdCo31 are indeed responsible for the columnar trait, they should be inherited by all the 'Wijcik' progeny showing a columnar phenotype. We verified this hypothesis using three standard and three columnar plants derived from a *Golden Delicious* x 'Wijcik' cross and found a strict correlation between presence of the insertion, increased expression of MdCo31 and columnar phenotype. Fig. 6A and 6B show the insert amplification and MdCo31 expression in standard and columnar progeny trees. Figure 6A illustrates that the specific amplification of insert sequence produced a PCR product for columnar progeny trees only. The primers used for amplification were 29f1 and JWI1r. Figure 6B shows the expression of MdCo31 in buds of standard and columnar trees. Expression levels are calculated based on three biological replicates and the standard deviations are indicated by error bars.

MdCo31 codes for a 2OG-Fe(II) oxygenase-like gene. 2OG-Fe(II) oxygenases catalyze the oxidation of organic substrates in plants (Prescott, 1996), They have been shown to be involved in the biosynthesis of gibberellins (Hedden, 2012), ethylene (John, 1997) and flavonoids (Prescott, 1996). The role of 2OG-Fe(II) oxygenases in gibberellin synthesis seems to be the most interesting regarding the phenotype of 'Wijcik'. Gibberellins are often found to be associated to dwarfism (Peng, 1999). In poplar, a columnar phenotype was observed in STUMPY, a tree resulting from an activation tagging screen and overexpressing a GA 2-oxidase, that has reduced levels of gibberellin (Busov, 2003).

The role of 2OG-Fe(II) oxygenases in the biosynthesis of flavonoids could provide another explanation for the effect of the increased expression of MdCo31 on the phenotype of 'Wijcik'. Flavonoids have been shown to inhibit auxin transport (Brown, 2001), a mechanism that is thought to be required for axillary bud outgrowth (Bennet, 2006; Przemyslaw, 2009).

Although a role of MdCo31 in gibberellin metabolism or flavonoid biosynthesis could possibly explain the phenotype of 'Wijcik', it is surprising that no clear homolog of MdCo31 was found in other plant species when a search using the amino acid sequence was performed.

Based on their putative function, a few other possible candidate genes were predicted inside the 393 kb Co region (see table 6), but more than 25 kb away from the insertion: a MYB protein (MdCo4) and three AP2 transcription factors (MdCo16, MdCo25 and MdCo26). Both MYB and AP2 transcription factors have been shown to be involved in plant developmental processes (Zhang, 2009; Guo, 2008; Yano, 2009; Kitomi, 2011).

**Table 6. Gene predictions from the Co region and their putative function**

| **Prediction** | **Contig** | **Start Position** | **Description** |
|---|---|---|---|
| MdCo1 | WiC1 | 4885 | Chromosome segregation ATPase |
| MdCo2 | WiC1 | 16370 | translocase inner membrane subunit |
| MdCo3 | WiC1 | 27606 | predicted protein |
| MdCo4 | WiC1 | 43209 | myb domain protein |
| MdCo5 | WiC1 | 60715 | DNA mismatch repair protein |
| MdCo6 | WiC1 | 72499 | Protein of unknown function, DUF647 |
| MdCo7 | WiC1 | 76626 | Protein of unknown function |
| MdCo8 | WiC1 | 79902 | actin depolymerizing factor |
| MdCo9 | WiC1 | 83253 | phosphate transporter |
| MdCo10 | WiC1 | 92605 | RNAseH-like protein |
| MdCo11 | WiC1 | 95489 | predicted protein |
| MdCo12 | WiC1 | 106812 | predicted protein |
| MdCo13 | WiC1 | 109222 | predicted protein |
| MdCo14 | WiC1 | 115595 | predicted protein |
| MdCo15 | WiC1 | 122386 | predicted protein |
| MdCo16 | WiC1 | 131567 | AP2 transcription factor |
| MdCo17 | WiC1 | 139082 | Retrotransposon protein |
| MdCo18 | WiC1 | 151296 | Retrotransposon protein |
| MdCo19 | WiC1 | 181283 | Retrotransposon protein |
| MdCo20 | WiC1 | 191207 | predicted protein |
| MdCo21 | WiC1 | 192786 | predicted protein |
| MdCo22 | WiC1 | 200156 | predicted protein |
| MdCo23 | WiC1 | 204431 | predicted protein |
| MdCo24 | WiC1 | 223258 | cysteine-rich RLK |
| MdCo25 | WiC1 | 236340 | AP2 transcription factor |
| MdCo26 | WiC1 | 242114 | AP2 transcription factor |
| MdCo27 | WiC1 | 246782 | predicted protein |
| MdCo28 | WiC1 | 258318 | Protein of unknown function |
| MdCo29 | WiC1 | 281163 | RING/U-box superfamily protein |
| MdCo30 | WiC1 | 286736 | predicted protein |
| MdCo31 | WiC1 | 297570 | 2OG-Fe(II) oxygenase family protein |
| MdCo32 | WiC1 | 301585 | Retrotransposon protein |
| MdCo33 | WiC1 | 307277 | RING/U-box superfamily protein |
| MdCo34 | WiC1 | 310294 | Vacuolar protein sorting protein |
| MdCo35 | WiC1 | 313533 | Tetratricopeptide repeat-like superfamily protein |
| MdCo36 | WiC1 | 323260 | Autophagy protein 9 |
| MdCo37 | WiC1 | 335884 | predicted protein |
| MdCo38 | WiC1 | 339324 | predicted protein |
| MdCo39 | WiC1 | 344289 | nodulin MtN21 /EamA-like transporter family protein |
| MdCo40 | WiC1 | 348746 | predicted protein |
| MdCo41 | WiC2 | 3305 | formyltetrahydrofolate synthetase |
| MdCo42 | WiC2 | 8297 | predicted protein |
| MdCo43 | WiC2 | 27835 | cysteine-rich RLK |

Although the expression of such genes is unlikely influenced by such a distant insertion, we measured the expression of these transcription factors. The transcript levels were found to be very low, and no differences between columnar and standard trees were found. These results seem to exclude a role for these genes in controlling columnar growth in apple.

In summary, this invention is based on the identification of an insertion of 1956 bp in the Co region of 'Wijcik'. The presence of the insertion was shown to be correlated with a high expression of MdCo31 in young buds of 'Wijcik' and of all the columnar progeny or transformed plants tested. These results strongly suggested that MdCo31 is the gene responsible for the columnar phenotype of 'Wijcik' which has been confirmed by the columnar tree architecture of plants which have been modified introducing the polynucleotide according to the invention. How the insertion found in 'Wijcik' influences the expression of MdCo31 remains unclear, but it is known that DNA mobile elements can affect the expression of neighboring genes in several ways. For example, the silencing machinery that is used to keep transposons under control can have an effect on the expression of neighboring genes (Slotkin, 2007). In addition, DNA mobile elements can contain *cis*-elements themselves or disrupt existing regulatory elements at their insertion site (Feschotte, 2008; Girard, 1999).

Interestingly, the effect of the insertion on the expression of MdCo31 seems to be tissue specific and the expression of the genes comprised between the insertion and MdCo31 was shown to be unaffected.

### Genetic transformation of plants

The genetic transformation of plants with the polynucleotide (gene MdCo31) according to the invention using a promoter has in principle been described above.

Some more specific methods for the genetic transformation are described in the following.

### Transformation of Arabidopsis

For example, to transform Arabidopsis thaliana the method described by Clough and Bent (1998) can be adopted.

An example for a specific transformation is the following procedure:

The first step is the electrotransformation of Agrobacterium with a plasmid that has been replicating in E. coli: the host Agro strain is streaked out from the -80°C onto the appropriate media (e.g., GV3101 onto YEP-Gen50). It will take 2-3 days to establish good size single colonies. Agro does not seem to last more than a month or two on selectable media plates in the fridge so it is wisest to always use a freshly streaked culture. Competent Cells: Liquid Culture Method; Additional items needed: ∼2500ml of ice-cold sterile (autoclaved) dH₂O and ice-cold 10% glycerol. A single colony of Agro is picked into ∼5ml YEP. It is grown overnight at 28°C. 1000ml of YEP are inoculated with the 5ml overnight culture. It is grown overnight (or until the OD600 is ∼1.5). The cells are chilled by placing the flask into an ice bath. The cells are spinned down at 2,500 X g (∼4200 RPM in SLA1500 rotor) for 5min at 4°C. The cells are resuspended in 1000ml ice cold sterile dH2O. The cells are spinned down 10,000 X g (∼8500 RPM in SLA1500 rotor) for 15min at 4°C. It is repeated with a second 1000ml volume of ice-cold sterile dH2O. It is repeated with 500ml ice-cold sterile dH₂O. It is resuspended in 20ml ice-cold sterile 10% glycerol. The cells are transferred to an Oak Ridge tube. The cells are spinned down at 10,000 X g (∼9000 RPM in SS34 rotor) for 15min at 4°C. They are resuspended in 2ml ice-cold sterile 10% glycerol. It is aliquoted into convenient volume (50 - 125µl). The tubes are *Quick-freezed in liquid N₂. The cells are stored at -80°C.

Competent Cells: Plate Method: a single colony is picked up and resuspended in 100 - 200 µL of sterile T10E0.1, H₂O or media. This suspension is plated onto a fresh media plate containing the appropriate antibiotic. It is grown for ∼48h. Using sterile technique, the cells are scraped off with a loop and resuspended into 1ml sterile dH₂O in a microfuge tube. The cells are spinned down (30 - 60 sec in the microfuge) and decanted off s/n, keeping the cells. The washing of the Agro cells is repeated with 1ml volumes of sterile dH₂O for several additional resuspension/decantation cycles. After the final washing and decantation, the cells are resuspended in a convenient volume (e.g., 50µL volume per electrotransformation is good). Electroporation: the DNA preferably should be cleaned up, e.g., CsCl, Qiagen (or some other Company's cleaning kit) or phenol extracted mini-prep DNA. ∼50µl Agro cells are electroporated with ∼1µl plasmid DNA from E. coli. The cells are outgrown in antibiotic free medium for 1-3 h (e.g. 1.5ml microfuge tubes). Afterward the cells are simply spinned down in a microfuge to concentrate them. Since the objective is to just obtain a single colony of the transformed construct it really makes no sense to plate serial dilutions. Therefore after outgrowing and pelleting the cells, a loop is simply flamed and the transformation mix is streaked onto selectable medium. The transformation mix is plated onto an appropriate selectable media plate (usu. YEP-Gen50Strep300, YEP-Gen50Spec100 or YEP-Gen50Kan50). It is grown for 2-3 days. Advantageously, a glycerol stock of this construct is made for future needs. Note: in a pinch and if you only have one or a few constructs to transform, a single colony can be picked of a plate and prepared for transformation (i.e., skipping the first steps).

In the following we describe the Agro growth and preparation: a single colony of the Agro transformed with the plasmid is picked into 5ml YEP containing the appropriate antibiotic. It is grown overnight. 250ml of YEP containing the appropriate a/b are inoculated with the 5ml grown overnight. It is grown overnight. The cells are spinned down at 9000 rpm in the Sorval SLA1500 for 5 min. The spent media is decanted into bleach or autoclave. The Agro cells are resuspended in 250ml 5% sucrose / 0.05% Silwet-L77.

In the following we describe the growth of *Arabidopsis thaliana.* Each lab strain has a different time to bolting (flowering). If plants are being grown for transformation; i.e., floral dipping, each strain will be at the optimum accordingly; i.e., WS2 is at its optimum three weeks after sowing when grown at 22°C constant temp, 18h day and 6h night. Nø2 is 7d to 10d later; RLD takes an additional two weeks, i.e., 3 weeks after WS2, to reach the appropriate stage for dunking. Pots are prepared with soil fitted with fiberglass netting secured with a rubber band. The soil netting prevents soil escaping from the pot when it is inverted for dunking the plants in the Agro solution; this step is not necessary otherwise. Seed is sown such that you end up with 10 - 20 plants per pot. (One way to do this is to make a suspension of Arabidopsis seed in 0.1% phytagar and then to pipette the seed onto the netting. Make sure the seed contact the soil, e.g., use a squirt bottle to ensure the seed are through the netting. There are approximately 50 seeds per mg). The pots are placed in a green tray. The tray is filled with about 1 inch of tap distilled water. It is covered with plastic wrap until the plants are visible, ∼3-5d. The plants are placed in a growth chamber on the bottom shelf. Florescent lamps are the best. If one high-pressure sodium lamp (essential to induce flowering) and one metal halide lamp (for vegetative growth) are being used then when the plants are placed on the bottom shelf the light intensity works out to 100 µMm-2min-1. Supposedly Arabidopsis don't like HPS. Note: if the seed are fresh and/or have not been completely dried down, they will need to be stratified. To do this either imbibe the seed and then place them in the refrigerator (in a microfuge tube) or, alternatively put the pots and trays into a cold room for 3 or 4d. Thin plants to 10 - 20 per pot before they get too crowded. The day before dunking, it has to be made sure that the plants are well watered.

In the following an Arabidopsis dunking procedure is illustrated: the plants to be transformed are brought from the growth chamber into the lab. The pot is inverted and the inflorescences (flowers) and leaves of the plants are dipped into the bacterial suspension (a 1000ml plastic beaker works well for this); it is swirled for about 5 sec. More 5% sucrose / 0.05% Silwet-L77 is added if needed (250ml is a convenient volume to work with because it fits in the 250ml centrifuge bottle). Several pots can be successively dipped in the same Agro suspension. The Agro is drained from the plants. The plants are laid on their side in the tray and the tray is covered with plastic wrap and placed in low light. It is lefty overnight. The next day the plastic wrap is removed and the plants are set upright. The plants are returned to the growth chamber. The plants are not watered again until they show signs of needing water, ∼5 d. Otherwise grow and harvest seed as normal.

For the continued growth and maintenance of plants follow the following steps: Generally when transgenic plants are created via the floral dip method the plants are growing in 4-inch pots. Once the plants are unable to support their inflorescence, a single stake (hyacinth stake) is put into the pot and the string is used to tie up all the plants in that pot. This essentially prevents cross contamination between adjacent pots of plants. Once the plants have all set seed and start to show signs of senescence they may be brought into the lab to dry down; i.e., they no longer require light to finish setting seed. For seed harvesting the plants should essentially be bone dry. Stake and string are removed. The plant is placed on top of a white piece of paper and your fingers are run up the stem to strip the seeds and siliques off of the plant and onto the piece of paper below. The siliques are scrunched one additional time to remove any remaining seed from the siliques. With another white piece of paper below, the seed is passed through a nylon mess to remove all debris.

This process is repeated one or two additional times until a relatively clean preparation of seed is obtained. To prevent cross contamination during seed harvest between each successive transgenic plant seed isolation the lab counter top area & paper is always thoroughly brushed and the mesh is shaken out. The seed is stored in a 1.5ml microfuge tube. They are quite stable at RT. For the plating and screening for transgenic Arabidopsis, firstly, GB5 plates containing the appropriate antibiotic are prepared. The successive seed sterilization** is done following the steps of: a) transferring a portion of the seed harvest to a second microfuge tube (1mg ∼50 seed); b) adding 1ml of freshly prepared H₂O/Bleach/Triton X-100 to the seeds; c) vortexing well; d) shaking for 10 - 20 min^{##}; e) vortexing well a second time; f) quick spinning; g) aspirating (or decanting) off H₂O/bleach/triton X-100 soln.; h) adding ∼1ml sterile H₂O; i) vortexing, quick spinning and aspirating off liquid; j) repeating seed washing with sterile H₂O two additional times; k) adding ∼1ml sterile 0.1% phytagar to seeds and plate; l) parafilming the plates and placing them under lights^^. The transfer of plants from tissue culture follows the following steps: as soon as possible the transgenic plants are removed from the antibiotic containing media plates and transferred to soil. The plants are gently removed from the agar surface. It might help to first loosen the agar surrounding the plants using forceps and then give the plant a gentle tug (Note that in lower concentrations of phytagar; e.g., ∼0.8%, the roots tend to penetrate the agar whereas in higher concentrations; e.g., ∼1.0% the roots tend to grow more on the surface.). If the objective is to grow numerous independent transgenic plants in very close proximity, then single individual plants are transferred in an individual cell in one of the cell packs (these are like what you buy bedding plants from a nursery in; they come in all sizes: 6 pack 8 packs etc). The newly transplanted plants are covered with plastic wrap. This is left on for several days until visible signs of growth from the transplants. To prevent cross contamination of seed between adjacent plants, each plant is tied to a hyacinth stake once large enough. The following solutions, media and other substances are needed: a) YEP 1L: yeast extract 10g; peptone 10g; NaCl 5g; dH₂O to 1L; Agar (for plates only) 15g; A/C; cooling to ∼50°C; adding of appropriate quantity of antibiotic. For the antibiotics: each needed antibiotic is prepared by dissolving it in the appropriate solvent (the following are all water soluble except*): antibiotic stock conc for 10ml of stock final conc in media: Gen 50mg/ml 0.5g 50µg/ml; kanamycin 50mg/ml 0.5g 50µg/ml; hygo 50mg/ml 0.5g 15-50µg/ml; Rif* 50mg/ml 0.5g 50µg/ml (in DMSO); Strep 300mg/ml 3g 10µg/ml E. coli, 300µg/ml Agro; spectinomycin 100mg/ml 1g 100µg/ml Agro; timenton 300mg/ml 3g 300µg/ml Arabidopsis T1, filter-sterilization a/b using syringe and 0.2µ filter unit, adding a/b to media after it has cooled to ∼50°C. Storage of a/b at -20°C.

0.1% phytagar: phytagar 0.1g; dH₂O 100ml; A/C. 5% sucrose / 0.05% Silwet-L77: dH₂O 250ml; sucrose 12.5g; Silwet-L77 125µL Silwet-L77; there is no need to sterilize if used the same day. Gamborg's B5 medium (GB5): GB5* 23.2g; MES 0.5g; dH₂O to 1 L; pH to 5.7; phytagar 8-10g^{#}; A/C; cooling to ∼50°C; adding appropriate quantity of antibiotic. H2O/bleach/triton X-100 soln^: dH₂O 50%; bleach 50%; triton X-100 0.2%; prepare fresh. Add the Triton to the dH₂O first and then apply heat (or heat the water and then add the triton), cool, then add the bleach (supposedly heat reduces the effectiveness of the bleach).

### NOTES:

*GB5 comes either with- or without- sucrose. The above recipe is based on the assumption that sucrose is present in the GB5 formulation. If it is straight GB5, then only 3.2g of the GB5 is required but then the media must be augmented with 20g of sucrose.
^{#}0.8% phytagar results in a soft surface which the plant roots will more than likely penetrate whereas 1.0% phytagar results in a less permeable surface which causes the roots to grow along the surface of the phytagar.
^ The concentrations of the bleach and Triton are not absolutely critical. Some people use 30% bleach and 0.02% Triton X-100. I like 50% bleach and 0.2% Triton X-100.
**Although the addition of the 0.1% phytagar and the plating of the seeds is performed in the laminar flow hood, all other sterilization and washing operations are performed at the bench.
##Don't leave the seeds in contact with the Bleach/Triton X-100 solution too long (about 1 hour). This solution will eventually kill the embryo.
^^If the seed are fresh, refrigerate (to vernalize) either the seed after the last washing step or the plates for 3-4d.

These are recombinant Agro cells; they must be killed either by autoclaving or by mixing with bleach.

For the vacuum infiltration see Bechthold et al. (1993); for dipping/dunking: see Bent and Clough and desfeux et al. Regarding GV3101 refer to: Koncz et al. (1986).

### Transformation of poplar plants

For example, for transformations of Hybrid aspen (Populus tremula × P. tremuloides)/clone 51 (Finnish clone) the following mediums are used:
Callus production medium (MS1): ½ MS (salts and vitamins, Duchefa M0222), 2% sucrose, 0.5 µM BAP + 4 µM 2,4-D; 0.7% agar, pH 5,6
Callus selection medium: MS1 (callus production medium, MS1) + antibiotic for selection + 250 mg/l claforan
Regeneration medium (MS2): ½ MS (salts and vitamins, Duchefa M0222), 2% sucrose, 0.1 µM TDZ, 0.7% agar, pH 5.6 + antibiotic for selection (+ 250 mg/l claforan)
MS3 medium: ½ MS (salts and vitamins, Duchefa M0222), 100 mg/l myo-Inositol, 2.85 µM IAA pH 5.6; agar (0.8%)

The transformation can be carried out according to the following protocol:

### Explants preculture (2)-3d before transformation

- Stem segments are cut into 3 to 5 mm pieces under sterile conditions, without let them dry, ½ MS + 2% sucrose solution is used on plate and the cut stem segments are transferred immediately to this solution
- approximately 300 (or more) stem segments are cut for each transformed construct to ensure formation of successful amount of independent transgenic lines (=6 to 10)
- explants are transferred into callus production medium plates (approximately 20 explants/plate), it is sealed with parafilm, and incubated in vitro under light (e.g. growth cabin) for 3 days. Nilsson et al. 1992 did not preculture, but it increases transformation efficiency most likely.

### Agrobacterium culture (GV3101 pMP90; start this at the same time as explant preculture):

- It is started with 5ml LB + appropriate antibiotics (e.g. with rifampicin 20 mg/l, gentamicin 30 mg/l [for Agro], kanamycin or spectinomycin 100 mg/l [depending on vector]), culture overnight at +28°C shaker
- It is continued with 50ml culture (LB + antibiotics, different dilutions for example 1 ml to 50ml and less are made), overnight +28°C shaker
- Next day OD600 is measured and when it reaches 0.3-0.8 (preferably at least 0.5) the culture is transferred under fume to sterile centrifuge tube/bottle, centrifugation for 10 min at 3000 rpm*
- the supernatant is poured off, ½ MS+ 2% sucrose solution is added to final density around 0.5 (OD600)
- acetosyringone is added to final concentration 20 µM, it is incubated for 30-60 min with gentle shaking (e.g. at RT, table shaker 60 rpm)
- explants are transferred to Agro solution and incubated 30 min to 4h (e.g. at RT, in light table shaker 60 rpm)
- explants are transferred to the callus production medium (plates without selection) for 2d, in light (e.g. in vitro growth cabin)
* (it is handy to use sterile 50ml Falcon tube. After incubation with acetosyringone explants are just added into tube and incubation is continued).

### Explants washing

- After 2d co-culture with Agro washing is started.
- explants are transferred for example to sterile decanter class containing ½ MS+ 2% sucrose solution, it is rinsed 3 times with MS solution (such a volume that all the explants will be covered with liquid, for rinsing plenty [100 to 300ml] may be used but 50ml is enough when vancomycin and claforan is used to save vancomysin)
- the solution is removed and fresh ½ MS+ 2% sucrose solution + 300 mg/l vancomycin and 500 mg/l claforan is added (it is preferred to use fresh vancomycin solution or only few days old solution), it is incubated twice at least 15 min at RT with gentle shaking
- explants are plotted quickly to sterile filter paper and transferred to the callus selection medium (callus production medium + transgene antibiotic [60 mg/l kanamycin or 15 mg/l hygromycin depending on vector; the hygromycin concentration may be decreased to 5 mg/l if callus is suffering for selection and 250 mg/l claforan for Agro)
- the plates are incubated in light (e.g. in vitro growth cabin; refers normal poplar tissue culture conditions as for vials, growth chamber is set to 18h light/6h dark, 20°C). Nilsson et al. 1992 did this also in light for T89, but dark conditions are sometimes also used
- explants are transferred about every 2 to 3 weeks to the fresh plate (e.g. first transfer after 2 weeks and then every third weeks)
- It takes about 1-2 months to see first callus structures (only callus formed, not shoots; compare Nilsson et al. 1992 with both callus and shoots), callus structures are transferred (when they are about 3-5 mm) to regeneration medium (MS2) 0.1 µM TDZ + antibiotic for selection (claforan must not be added anymore if Agro contamination is not a problem)
- After shoot initiation plantlets are transferred to MS3 medium.

### Transformation of tomato plant

Tomatoes can be transformed using the following indications.

### Plants

1. Seeds are sterilized in Moneymaker 20' in 1% NaOCl (83ml 12% NaOCl per liter water)
2. It is rinsed 4 times with water.
3. Seeds are sown on GEM (germination medium); 25 seeds per container.
4. It is incubated ±3 days at 4°C, ±10 days at 24°C.

### Agrobacterium

1. A single colony of Agrobacterium tumefaciens (e.g. AGL1+extra virG) is streaked in 2ml LB+antibiotics, grown at 28°C for 2 days.
2. 0,5ml are diluted in 10ml LB+antibiotics, and grown overnight at 28°C.
3. Agrobacterium cultures are centrifugated 15' at 3000 rpm.
4. Resuspension in SIM+acetosyringon (100 µl/l), dilution to OD600 0,5 and 10ml are put in a fresh 50ml tube. Incubation overnight at 25°C in the dark.
5. Next day: centrifugation 15' at 3000 rpm, resuspension in 20ml MSO+acetosyringon (1 ml/l). OD600 is 0,25.

### Transformation

1. 10ml MSO+acetyringon are pipetted in a petri dish.
2. Cotelydons are taken, the tip is cut off and the cotelydon is cut in 2 transversal pieces. The leaf pieces are let float upside down on the MSO medium. 1 pot gives 100 leaf pieces, 200 pieces fit in 1 petri dish.
3. 10ml agrobacterium solution are added, final OD600 is 0,125. Incubation for 10-15' with occasional swirling.
4. Explants are blotted on sterile filter paper and placed with abaxial side down (=upside down) on GCF10+acetosyringon (1ml/l). First a round sterile filter paper is put on the medium, leave pieces are placed on top of this.
5. It is incubated in a 25°C incubator in the dark for 48 hours.
6. After 2 days of incubation the explants are transferred to plates containing GCF10+timentin300 and kanamycin100. It is incubated at 25°C in the light (20-25 leaf pieces per petri dish).
7. It is changed to fresh GCF10+tim+kan after 3 weeks.
8. Calli are excised from explants and transferred to higher petri dishes containing GCF11+ timentin300 and kanamycin100.
9. Once shoots are big enough, they are transferred to rooting medium MS30B5+ kanamycin100.

### Mediums

GEM: MS ½ conc (2,2 g/l), sucrose (10 g/l), pH 5,8, Daishin agar (8 g/l).
SIM: sodium citrate (20 mM), sucrose (20 g/l), pH 5,5)
MSO: MS salts (4,3 g/l), MS salts=MS without vitamins; thiamine (0,4 mg/l), myo-inositol (100 mg/l), sucrose (30 g/l), pH 5,8.
GCF10: MS salts (4,3 g/l), vitamins Nitsch (108,73 mg/l), sucrose (30 g/l), pH 5,8, agar (8 g/l), zeatine riboside (1,5 mg/l), IAA (0,2 mg/l).
GCF11: MS salts (4,3 g/l), vitamins Nitsch (108,73 mg/l), sucrose (30 g/l), pH 5,8, agar (8 g/l), zeatine riboside (1,9 mg/l).
MS30B5: MS salts (4,3 g/l), vitamins B5 (112,0 mg/l), sucrose (30 g/l), pH 5,8, agar (8 g/l).
Acetosyringon: stock = 200 mM in DMSO.

Addition of zeatin riboside, IAA, timentin, acetosyringon and antibiotics after autoclaving. Addition of acetosyringon in liquid medium just before use.

### References

Almasia N. I., Narhirñak V., Hopp H. E., Vazquez-Rovere C. (2010). Isolation and characterization of the tissue and development-specific potato snakin-1 promoter inducible by temperature and wounding. Electron. J. Biotechnol. vol.13 no.5.
Bai, T., Y. Zhu, et al. (2012). "Fine genetic mapping of the Co locus controlling columnar growth habit in apple." Molecular Genetics and Genomics 287(5): 437-450.
Baldi, P., P. J. Wolters, et al. (2012). Genetic and physical characterisation of the locus controlling columnar habit in apple (Malus × domestica Borkh) Mol Breeding (published online, DOI 10.1007/s11032-012-9800-1).
Barritt, B. H. (1992). Intensive orchard management. Washington USA, Good Fruit Growers.
Bechtold, N., Ellis, J., and G. Pelletier (1993) In planta Agrobacterium mediated gene transfer by infiltration of adult Arabidopsis thaliana plants. C.R. Acad Sci. Paris, Life Sciences 316: 1194-1199.
Bennetzen, J. L. (2000). "Transposable element contributions to plant gene and genome evolution." Plant Molecular Biology 42(1): 251-269.
Bent, A.F. and S.J. Clough in "Agrobacterium germ-line transformation: Transformation of Arabidopsis without tissue culture." In: Plant Molecular Biology Manual (Second Edition), S.B. Gelvin and R.A. Schilperoort, eds. (Kluwer Academic Pub., Dordrecht, The Netherlands).
Botton, A., G. Eccher, et al. (2011). "Signaling pathways mediating the induction of apple fruitlet abscission." Plant Physiology 155(1): 185-208.
Busov, V. B., R. Meilan, et al. (2003). "Activation tagging of a dominant gibberellins catabolism gene (GA 2-oxidase) from poplar that regulates tree stature." Plant Physiology 132(3): 1283-1291.
Chevreux, B., T. Wetter, et al. (1999). Genome sequence assembly using trace signals and additional sequence information. German Conference on Bioinformatics 45-56.
Christensen A.H., Sharrock R.A., Quail P.H. (1992) Maize polyubiquitin genes: structure, thermal perturbation of expression and transcript slicing, and promoter activity following transfer to protoplasts by electroporation. Plant Mol Bioi 18:675-689.
Clough, St. J., Bent, A. F. (1998). "Floral dip: a simplified method for Agrobacterium-mediated transformation of Arabidopsis thaliana". The Plant Journal 16(6): 735-743.
Conner, P. J., S. K. Brown, et al. (1997). "Randomly amplified polymorphic DNAbased genetic linkage map of three apple cultivars." Journal of American Society for Horticultural Science 122: 350-359.
Depicker A., Stachel S., Dhaese P., Zambryski P., Goodman H. M. (1982) Nopaline synthase: the transcript mapping and DNA sequence. J Mol Appl Genet 1: 561-573.
Desfeux, C., Clough, S. J., and Bent, A. F. Female reproductive tissue are the primary target of Agrobacterium-mediated transformation by the Arabidopsis floral-dip method. Plant Physiol. 123: 895-904.
Elliott K.A. and Shirsat A.H. (1998). Promoter regions of the extA extensin gene from Brassica napus control activation in response to wounding and tensile stress. Plant Molecular Biology 37: 675-687, 1998.
Feschotte, C. (2008). "Transposable elements and the evolution of regulatory networks." Nature Reviews Genetics 9(5): 397-405.
Fisher, D. V. (1969). "Spur-type strains of 'McIntosh' for high density planting." British Columbia Fruit Grower's Association Quart. Rep. 14: 3-10.
Flachowsky, H., C. Hattasch, et al. (2010). "Overexpression of LEAFY in apple leads to a columnar phenotype with shorter internodes." Planta 231(2): 251-263.
Franck A., Guilley H., Jonard G., Richards K., Hirth L. (1980) Nucleotide sequence of cauliflower mosaic virus DNA. Cell 21:285-294.
Girard, L. and M. Freeling (1999). "Regulatory changes as a consequence of transposon insertion." Developmental Genetics 25(4): 291-296.
Guo, M., J. Thomas, et al. (2008). "Direct repression of KNOX loci by the ASYMMETRIC LEAVES1 complex of Arabidopsis." The Plant Cell 20(1): 48-58.
Hedden, P. and S. G. Thomas (2012). "Gibberellin biosynthesis and its regulation." Biochemical Journal 444(1): 11-25.
Hemmat, M., N. F. Weeden, et al. (1997). "A DNA marker for columnar growth habitat in apple contains a simple sequence repeat." Journal of American Society for Horticultural Science 122(347-349).
Hu Y., Chen B., Ni T., Li N., Lin Z. (2003). Promoter of the roIC gene of Agrobacterium rhizogenes can be strongly regulated in glandular cell of transgenic tobacco. Mol Biotechnol. 24:121-6.
Jacobsen E. & Schouten H. J. (2008). Cisgenesis, a New Tool for Traditional Plant Breeding, Should be Exempted from the Regulation on Genetically Modified Organisms in a Step by Step Approach. Potato Research 51:75-88.
John, P. (1997). "Ethylene biosynthesis: The role of 1-aminocyclopropane-1-carboxylate (ACC) oxidase, and its possible evolutionary origin." Physiologia Plantarum 100(3): 583-592.
Kent, W. J. (2002). "BLAT--the BLAST-like alignment tool." Genome Research 12(4): 656-664.
Kim, M. Y., K. J. Song, et al. (2003). "Development of RAPD and SCAR markers linked to the Co gene conferring columnar growth habit in apple (Malus pumila Mill.)." The Journal of Horticultural Science & Biotechnology 78(4): 6.
Kitomi, Y., H. Ito, et al. (2011). "The auxin responsive AP2/ERF transcription factor CROWN ROOTLESS5 is involved in crown root initiation in rice through the induction of OsRR1, a type-A response regulator of cytokinin signaling." The Plant Journal 67(3): 472-484.
Koncz, Csaba, and Jeff Schell (1986) The promoter of TL-DNA gene 5 controls the tissue-specific expression of chimaeric genes carried by a novel type of Agrobacterium binary vector. Mol. Gen Genet. 204:383-396.
Krost, C., R. Petersen, et al. (2012). "The transcriptomes of columnar and standard type apple trees (Malus x domestica) - A comparative study." Gene 498(2): 223-230.
Lapins, K. O. (1976). "Inheritance of compact growth type in apple." Journal of American Society for Horticultural Science 101: 133-135.
Lespinasse, J. M. and J. F. Delort (1993). "Regulation of fruiting in apple role of the course and crouwned brindles." Acta Horticultura 349: 7.
Li, J. and R. Yuan (2008). "NAA and ethylene regulate expression of genes related to ethylene biosynthesis, perception, and cell wall degradation during fruit abscission and ripening in 'Delicious' apples." Journal of Plant Growth Regulation 27(3): 283-295.
Majoros, W. H., M. Pertea, et al. (2004). "TigrScan and GlimmerHMM: two open source ab initio eukaryotic gene-finders." Bioinformatics 20(16): 2878-2879.
Marchler-Bauer, A., S. Lu, et al. (2011). "CDD: a Conserved Domain Database for the functional annotation of proteins." Nucleic Acids Research 39 (Database issue): D225-229.
Medberry S. L., Lockhart B. E., Olszewski N. E. (1990). Properties of Commelina yellow mottle virus's complete DNA sequence, genomic discontinuities and transcript suggest that it is a pararetrovirus. Nucleic Acids Res. 18:5505-13.
Miller, S. A., F. D. Broom, et al. (2001). "Effects of leader pruning on vine architecture, productivity and fruit quality in kiwifruit (Actinidia deliciosa cv. Hayward)." Scientia Horticulturae 91(3-4): 189-199.
Moriya, S., H. Iwanami, et al. (2009). "Development of a marker-assisted selection system for columnar growth habit in apple breeding." Journal of Japanese Society for Horticultural Science 78: 279-287.
Moriya, S., K. Okada, et al. (2012). "Fine mapping of Co, a gene controlling columnar growth habit located on apple (Malus x domestica Borkh) linkage group 10." Plant Breeding 131: 641-647.
Mudunuri, S. B. and H. A. Nagarajaram (2007). "IMEx: Imperfect Microsatellite Extractor." Bioinformatics 23(10): 1181-1187.
Nilsson et al. 1992. Spatial pattern of cauliflower mosaic virus 35S promoter-luciferase expression in transgenic hybrid aspen trees monitored by enzymatic assay and non-destructive imaging. Transgenic Research 1: 209-220. Ning, Z., A. J. Cox, et al. (2001). "SSAHA: a fast search method for large DNA databases." Genome Research 11(10): 1725-1729.
Peng, J., D. E. Richards, et al. (1999). "'Green revolution' genes encode mutant gibberellin response modulators." Nature 400(6741): 256-261.
Pfaffl, M. W. (2001). "A new mathematical model for relative quantification in realtime RT-PCR." Nucleic Acids Research 29(9): e45.
Prescott, A. G. and P. John (1996). "DIOXYGENASES: Molecular structure and role in plant metabolism." Annual Review of Plant Physiology and Plant Molecular Biology 47: 245-271.
Rhee, S. Y., W. Beavis, et al. (2003). "The Arabidopsis Information Resource (TAIR): a model organism database providing a centralized, curated gateway to Arabidopsis biology, research materials and community." Nucleic Acids Research 31(1): 224-228.
Robinson, J. T., H. Thorvaldsdottir, et al. (2011). "Integrative genomics viewer." Nature Biotechnology 29(1): 24-26.
Schouten H. J., Krens F. A., Jacobsen E. (2006) Cisgenic plants are similar to traditionally bred plants: international regulations for genetically modified organisms should be altered to exempt cisgenesis. EMBO Rep.7:750-3.
Slotkin, R. K. and R. Martienssen (2007). "Transposable elements and the epigenetic regulation of the genome." Nature Reviews Genetics 8(4): 272-285.
Thorp, T. G. and B. Stowell (2001). "Pruning height and selective limb removal affect yield of large 'Hass' avocado trees." HortScience 36(4): 4.
Tian, Y., C. Wang, et al. (2005). "Mapping Co, a gene controlling the columnar phenotype of apple, with molecular markers." Euphytica 145: 181-188.
Tobutt, K. R. (1985). "Breeding columnar apples at East Malling." Acta Horticultura 159: 63-68.
Velasco, R., A. Zharkikh, et al. (2010). "The genome of the domesticated apple (Malus x domestica Borkh)." Nature Genetics 42(10): 833-839.
Wang M. B., Boulter D. and Gatehouse J. A. (1992). A complete sequence of the rice sucrose synthase-1 (RSsl) gene. Plant Molecular Biology 19: 881-885.
Wünsche, J. N. and A. N. Lakso (2000). "The relationship between leaf area and light interception by spur and extension shoot leaves and apple orchard productivity." HortScience 35: 5.
Yano, R., Y. Kanno, et al. (2009). "CHOTTO1, a putative double APETALA2 repeat transcription factor, is involved in abscisic acid-mediated repression of gibberellins biosynthesis during seed germination in Arabidopsis." Plant Physiology 151(2): 641-654.
Zhang, Y., G. Cao, et al. (2009). "Characterization of Arabidopsis MYB transcription factor gene AtMYB17 and its possible regulation by LEAFY and AGL15." Journal of Genetics and Genomics 36(2): 99-107.
Zhang, Y., J. Zhu, et al. (2012). "Characterization of transcriptional differences between columnar and standard apple trees using RNA-Seq." Plant Molecular Biology Reporter: 1-9.
Zhu, Y. D., W. Zhang, et al. (2007). "Evaluation of inter-simple sequence repeat analysis for mapping the Co gene in apple (Malus pumila Mill.)." The Journal of Horticultural Science & Biotechnology 82(3): 5.
Zuo J. and Chua N. H. (2000). Chemical-inducible systems for regulated expression of plant genes. Current Opinion in Biotechnology 11:146-151.

### Sequence listing free text:

The SEQ ID NOs 3-129 concern (forward and reverse) primers with the respective primer denomination and in the case of SEQ ID NOs 60-75 with reference to the respective gene and in the case of SEQ ID NOs 76-95 with reference to the respective SSR markers.

## Claims

1. An isolated polynucleotide, encoding a polypeptide having at least 90%, preferably at least 95%, more preferably at least 98% sequence identity to the amino acid sequence of SEQ ID No: 2, wherein % identity is calculated over the whole length of the amino acid sequence, wherein the polynucleotide encodes 20G-Fe(II) oxygenase-like gene.

2. The polynucleotide according to claim 1), wherein the polypeptide has the amino acid sequence of SEQ ID NO:2.

3. The polynucleotide of claim 1) wherein said polynucleotide has at least 90%, preferably at least 95%, more preferably at least 98% sequence identity to the nucleotide sequence of SEQ ID NO:1.

4. The polynucleotide according to claim 3), wherein said polynucleotide has the nucleotide sequence of SEQ ID NO:1.

5. A genetic construct comprising the polynucleotide of anyone of the claims 1) - 4).

6. An expression product of the polynucleotide according to anyone of the claims 1) - 4).

7. A vector comprising the polynucleotide according to anyone of the claims 1) -4).

8. A host cell transformed with the polynucleotide of anyone of the claims 1) - 4) to express said polypeptide.

9. A plant cell or plant transformed with the polynucleotide of anyone of the claims 1) - 4) to express said polypeptide, and wherein the expression of said polypeptide results in reduced branch and/or trunk growth, preferably in shorter axillary shoots in said transformed plant cell or plant.

10. A plant cell or plant according to claim 9), wherein the plant cell or plant is not of the mutant 'Wijcik'.

11. A plant, according to claims 9) and 10), preferably an apple tree, comprising, as a result of genetic transformation, a fragment of the chromosome 10 of the 'Wijcik' mutant of Malus x domestica Borkh that comprises a polynucleotide encoding a polypeptide which is at least 90%, preferably at least 95%, more preferably at least 98% identical to SEQ ID NO:2 or which is SEQ ID NO:2, wherein said plant has shorter axillary shoots than a control plant.

12. The plant according to anyone of the claims 9) - 11), wherein the expression of the polynucleotide is greater than the expression of the respective homologous native Co gene.

13. A method for producing a plant cell or plant with controlled tree architecture, the method comprising the steps of transformation of a plant cell or plant with the polynucleotide of one of the claims 1) - 4) and expression of said polypeptide encoded by said polynucleotide in the transformed plant cell or plant, and wherein said expression of said polypeptide reduces branch and/or trunk growth in said transformed plant.

14. A use of the polynucleotide according to one of the claims 1) - 4), the vector according to claim 7), the expression product according to claim 6), the genetic construct according to claim 5) or the host cell according to claim 8), to control branch and trunk growth in a plant, preferably in a plant selected from fruit trees, in particular apple trees, or ornamental trees wherein the controlled branch and trunk growth preferably results in a predefined number and length of axillary shoots.

15. A germ plasm of the plant according to anyone of the claims 9) to 12), preferably a seed or pollen, containing the polynucleotide of anyone of the claims 1) - 4).

## Patentansprüche

1. Isoliertes Polynukleotid, das ein Polypeptid mit wenigstens 90%, vorzugsweise wenigstens 95%, noch bevorzugter wenigstens 98% Sequenzidentität mit der Aminosäurensequenz von SEQ ID Nr:2 kodiert, wobei der Identitätsprozentsatz über die gesamte Länge der Aminosäurensequenz berechnet wird, wobei das Polynukleotid ein oxygenase-ähnliches 20G-Fe(II) Gen kodiert.

2. Polynukleotid gemäß Patentanspruch 1), wobei das Polypeptid die Aminosäurensequenz von SEQ ID Nr:2 hat.

3. Polynukleotid aus Patentanspruch 1), wobei das besagte Polynukleotid wenigstens 90%, vorzugsweise wenigstens 95%, noch bevorzugter wenigstens 98% Sequenzidentität mit der Nukleotidsequenz von SEQ ID Nr:1 hat.

4. Polynukleotid gemäß Patentanspruch 3), wobei das besagte Polynukleotid die Nukleotidsequenz von SEQ ID Nr:1 hat.

5. Genkonstrukt, das Polynukleotid aus einem jeden der Patentansprüche von 1) bis 4) umfassend.

6. Expressionsprodukt des Polynukleotids aus einem jeden der Patentansprüche von 1) bis 4).

7. Vektor, das Polynukleotid aus einem jeden der Patentansprüche von 1) bis 4) umfassend.

8. Wirtszelle, die mit dem Polynukleotid aus einem jeden der Patentansprüche von 1) bis 4) umgewandelt wurde, um das besagte Polypeptid auszudrücken.

9. Pflanzenzelle oder Pflanze, die mit dem Polynukleotid aus einem jeden der Patentansprüche von 1) bis 4) umgewandelt wurde, um das besagte Polypeptid auszudrücken, und wobei die Expression des besagten Polypeptids in einem verminderten Zweig- und/oder Stammwachstum resultiert, vorzugsweise in kürzeren Achselsprossen in der besagten, umgewandelten Pflanzenzelle oder Pflanze.

10. Pflanzenzelle oder Pflanze gemäß Patentanspruch 9), wobei die Pflanzenzelle oder Pflanze nicht von dem Mutanten 'Wijcik' stammt.

11. Pflanze gemäß den Patentansprüchen 9) und 10), vorzugsweise ein Apfelbaum, infolge von Gentransformation ein Fragment des Chromosoms 10 des 'Wijcik'-Mutanten von Malus x Domestica Borkh umfassend, welches ein Polynukleotid umfasst, das ein Polypeptid kodiert, welches wenigstens zu 90%, vorzugsweise wenigstens zu 95%, noch bevorzugter wenigstens zu 98% identisch ist mit SEQ ID Nr:2 oder das SEQ ID Nr:2 ist, wobei die besagte Pflanze kürzere Achselsprossen hat als eine Kontrollpflanze.

12. Pflanze gemäß eines jeden der Patentansprüche von 9) bis 11), wobei die Expression des Polynukleotids größer ist als die Expression des entsprechenden, homologen nativen Co-Gens.

13. Verfahren zur Produktion einer Pflanzenzelle oder Pflanze mit kontrollierter Stammbaumarchitektur, wobei das Verfahren die Schritte der Umwandlung einer Pflanzenzelle oder Pflanze mit dem Polynukleotid aus einem der Patentansprüche von 1) bis 4) und die Expression des besagten, durch das besagte Polynukleotid kodierten Polypeptids in der umgewandelten Pflanzenzelle oder Pflanze umfasst, und wobei die besagte Expression des besagten Polypeptids das Zweig- und/oder Stammwachstum in der besagten, umgewandelten Pflanze reduziert.

14. Verwendung des Polynukleodids gemäß eines der Patentansprüche von 1) bis 4), des Vektoren gemäß Patentanspruch 7), des Expressionsprodukts gemäß Patentanspruch 6), des Genkonstrukts gemäß Patentanspruch 5) oder der Wirtszelle gemäß Patentanspruch 8), um das Zweig- und Stammwachstum in einer Pflanze zu kontrollieren, vorzugsweise in einer aus Obstbäumen ausgewählten Pflanze, insbesondere in Apfel- oder Zierbäumen, wobei das kontrollierte Zweig- und Stammwachstum vorzugsweise zu einer vorbestimmten Anzahl und Länge von Achselsprossen führt.

15. Keimplasma der Pflanze gemäß eines jeden der Patentansprüche von 9) bis 12), vorzugsweise ein Samen oder Pollen, das das Polynukleotid aus einem jeden der Patentansprüche von 1) bis 4) enthält.

## Revendications

1. Un polynucléotide isolé, codant un polypeptide ayant une identité de séquence d'au moins 90%, préférablement d'au moins 95%, plus préférablement d'au moins 98% par rapport à la séquence d'acides aminés de SEQ ID NO:2, où l'identité de % est calculée sur toute la longueur de la séquence d'acides aminés, où le polynucléotide code un gène de type oxygénase 20G-Fe(II).

2. Le polynucléotide selon la revendication 1), où le polypeptide présente la séquence d'acides aminés de SEQ ID NO:2.

3. Le polynucléotide selon la revendication 1), où ledit polynucléotide présente une identité de séquence d'au moins 90%, préférablement au moins 95%, plus préférablement au moins 98% à la séquence nucléotidique de SEQ ID NO:1.

4. Le polynucléotide selon la revendication 3), où ledit polynucléotide présente la séquence nucléotidique de SEQ ID NO:1.

5. Une construction génétique comprenant le polynucléotide d'une quelconque des revendications de 1) à 4).

6. Un produit d'expression du polynucléotide selon l'une quelconque des revendications de 1) à 4).

7. Un vecteur comprenant le polynucléotide selon l'une quelconque des revendications de 1) à 4).

8. Une cellule hôte transformée avec le polynucléotide d'une quelconque des revendications de 1) à 4) pour exprimer ledit polypeptide.

9. Une cellule végétale ou plante transformée avec le polynucléotide d'une quelconque des revendications de 1) à 4) pour exprimer ledit polypeptide, et où l'expression dudit polypeptide entraîne une croissance réduite des branches et/ou du tronc, préférablement en pousses supplémentaires plus courtes dans ladite cellule végétale ou plante transformée.

10. Une cellule végétale ou une plante selon la revendication 9), où la cellule végétale ou plante n'est pas du mutant 'Wijcik'.

11. Une plante selon les revendications 9) et 10), préférablement un pommier, comprenant, comme résultat d'une transformation génétique, un fragment du chromosome 10 du mutant 'Wijcik' de Malus x domestica Borkh qui comprend un polynucléotide codant un polypeptide qui est égal à SEQ ID NO:2 pour au moins 90%, préférablement pour au moins 95%, plus préférablement pour au moins 98% ou qui représente SEQ ID NO:2, où ladite plante présente des pousses supplémentaires plus courtes qu'une plante de contrôle.

12. La plante selon l'une quelconque des revendications de 9) à 11), où l'expression du polynucléotide est plus grande que l'expression du relatif gène Co natif homologue.

13. Une méthode pour la production d'une cellule végétale ou plante avec une architecture arborescente contrôlée, la méthode comprenant les phases de transformation d'une cellule végétale ou plante avec le polynucléotide d'une quelconque des revendications de 1) à 4) et l'expression dudit polypeptide codé par ledit polynucléotide dans la cellule végétale ou plante transformée, et où ladite expression dudit polypeptide réduit la croissance des branches et/ou du tronc dans ladite plante transformée.

14. Une utilisation du polynucléotide selon l'une quelconque des revendications de 1) à 4), le vecteur selon la revendication 7), le produit d'expression selon la revendication 6), la construction génétique selon la revendication 5) ou la cellule hôte selon la revendication 8), pour contrôler la croissance des branches et du tronc dans une plante, préférablement dans une plante sélectionnée parmi des arbres fruitiers, en particulier pommiers, ou des arbres ornementaux où la croissance contrôlée des branches et du tronc entraîne préférablement un numéro et une longueur prédéfinis de pousses supplémentaires.

15. Un plasma germinatif de la plante selon l'une quelconque des revendications de 9) à 12), préférablement une graine ou un pollen, contenant le polynucléotide d'une quelconque des revendications de 1) à 4).
